# EUROPEAN PATENT SPECIFICATION

(11) **EP 2 411 006 B1**
(45) Date of publication and mention of the grant of the patent: **23.09.2020**
(21) Application number: 10756917.0
(22) Date of filing: 26.03.2010
(51) Int. Cl.: A61K 31/404, A61K 31/407, A61K 35/16, A61K 35/22, A61P 13/12, A61P 13/02, A61P 13/00

(54) **COMPOSITIONS AND METHODS FOR TREATMENT OF RENAL DISEASE**
ZUSAMMENSETZUNGEN UND VERFAHREN ZUR BEHANDLUNG VON NIERENKRANKHEIT
COMPOSITIONS ET MÉTHODES UTILISABLES DANS LE CADRE DU TRAITEMENT D'AFFECTIONS RÉNALES

(30) Priority: 26.03.2009 US 211051 P
(43) Date of publication of application: 01.02.2012
(73) Proprietor: Gupta, Ajay, Cerritos, CA 90703 (US)
(72) Inventor: Gupta, Ajay, Cerritos, CA 90703 (US)
(74) Representative: Lang, Christian
(86) International application number: PCT/US2010/028841
(87) International publication number: WO 2010/111599

(56) References cited:
- WO-A1-2008/124611
- WO-A2-00/27380
- WO-A2-96/24373
- BEAUFILS ET AL: "Nephroangiosclerose", EMC - NEPHROLOGIE, ELSEVIER, vol. 2, no. 3, 1 August 2005 (2005-08-01), pages 103-124, XP027827841, ISSN: 1638-6248 [retrieved on 2005-08-01]
- BIANCHI S ET AL: "Long-term effects of spironolactone on proteinuria and kidney function in patients with chronic kidney disease", KIDNEY INTERNATIONAL, vol. 70, no. 12, December 2006 (2006-12), pages 2116-2123, XP009160945, ISSN: 0085-2538
- PUIG ET AL: "Efficacy of Indapamide SR Compared With Enalapril in Elderly Hypertensive Patients With Type 2 Diabetes", AMERICAN JOURNAL OF HYPERTENSION, NATURE PUBLISHING GROUP, UNITED STATES, vol. 20, no. 1, 29 December 2006 (2006-12-29), pages 90-97, XP005818930, ISSN: 0895-7061, DOI: 10.1016/J.AMJHYPER.2006.05.018
- GAMBARDELLA S ET AL: "Regression of microalbuminuria in type II diabetic, hypertensive patients after long-term indapamide treatment", AMERICAN HEART JOURNAL, MOSBY- YEAR BOOK INC, US, vol. 122, no. 4, 1 October 1991 (1991-10-01), pages 1232-1238, XP022951770, ISSN: 0002-8703, DOI: 10.1016/0002-8703(91)90946-F [retrieved on 1991-10-01]
- MOTWANI.J.G.: 'Combining renin-angiotension-aldosterone system blockade with diuretic therapy for treatment of hypertension' JOURNAL OF RENIN-ANGIOTENSIN- ALDOSTERONE SYSTEM vol. 3, 2002, pages 72 - 78, XP009160965
- RACHMANI,R. ET AL.: 'The effect of spironolactone, cilazapril and their combination on albuminuria in patients with hypertension and diabetic nephropathy is independent of blood pressure reduction' DIABETIC MEDICINE vol. 21, no. 5, 2004, pages 471 - 475, XP009161130
- MANN JOHANNES F E ET AL: "Renal outcomes with telmisartan, ramipril, or both, in people at high vascular risk (the ONTARGET study): a multicentre, randomised, double-blind, controlled trial.", LANCET 16 AUG 2008, vol. 372, no. 9638, 16 August 2008 (2008-08-16), pages 547-553, ISSN: 1474-547X
- BAKRIS G L ET AL: "Treatment of microalbuminuria in hypertensive subjects with elevated cardiovascular risk: results of the IMPROVE trial.", KIDNEY INTERNATIONAL OCT 2007, vol. 72, no. 7, October 2007 (2007-10), pages 879-885, ISSN: 0085-2538
- BOMBACK ANDREW S ET AL: "The incidence and implications of aldosterone breakthrough.", NATURE CLINICAL PRACTICE. NEPHROLOGY SEP 2007, vol. 3, no. 9, September 2007 (2007-09), pages 486-492, ISSN: 1745-8331

## Description

### CROSS REFERENCE TO RELATED APPLICATIONS

This Application claims priority to United States Provisional Patent Application Serial No. 61/211,051, filed March 26, 2009.

### FIELD OF THE INVENTION

The invention relates to methods and compositions related to the treatment of subjects with proteinuria and/or hypertension (e.g., proteinuria and/or hypertension arising from primary renal disease or secondary to other conditions (e.g., diabetes, diabetic nephropathy, liver disease). Specifically, the invention relates to methods wherein an indoline (i.e., indapamide) is administered in combination with an anti-aldosterone agent (e.g., spironolactone and/or eplerenone).

### BACKGROUND OF THE INVENTION

Diabetes mellitus is one of the leading causes of morbidity and mortality in the United States because of its role in the development of ophthalamic, renal, neuropathic, and cardiovascular disease. These complications, particularly cardiovascular disease (∼50-75% of medical expenditures), are the major sources of expense and suffering for patients with diabetes mellitus. Approximately two thirds of people with diabetes die from heart disease or stroke. Men with diabetes face a 2-fold increased risk for coronary heart disease, and women have a 3-to 4-fold increased risk. In 1994, 1 of every 7 health care dollars in the United States was spent on patients with diabetes mellitus. The 2002 estimate for direct medical costs due to diabetes in the United States was $92 billion, with another $40 billion in indirect costs. Approximately 20% of Medicare funds are spent on patients with diabetes.

Diabetic nephropathy affects approximately 20-40% of all individuals with diabetes (American Diabetes Association (2009) Diabetes Care 32:S13-S61) and is the single most common cause of end-stage renal disease (ESRD) in the United States, leading to the need for dialysis. Risk for the development of diabetic nephropathy is directly related to the cumulative duration and severity of hyperglycemia (Wingard et al. (1993) Diabetes Care 16:1022-1025; herein incorporated by reference in its entirety), as well as the cumulative duration and severity of hypertension that commonly accompanies diabetes mellitus (Raile et al. (2007) Diabetes Care 30:2523-2528;) although other non-modifiable risk factors such as age and genetic background also influence that risk. This places individuals of African-American descent at particular risk, due to their disproportionately higher prevalence and severity of hypertension (Lopes et al.(2004) J. Clin. Hypertens. 5:393-401; The intensive control of both hyperglycemia and elevated blood pressure (BP) has clearly been shown to reduce the onset and severity of diabetic nephropathy. The pivotal Diabetes Complications and Control Trial (DCCT) in individuals with type 1 diabetes (N. Engl. J. Med. (1993) 329:977-986; and the United Kingdom Prospective Diabetes Study (UKPDS) in individuals with type 2 diabetes (Lancet (1998)352:837-853;) clearly established the benefits of intensive glycemic control for delaying the onset and worsening of diabetic nephropathy. Data from the UKPDS also clearly established the benefits of intensive BP control on delaying the progression of diabetes complications, including nephropathy (BMJ (1998) 317:703-713;).

Renal damage in diabetes, as with renal damage due to primary kidney disease, involves proteinuria of glomerular origin. Renal tubules of the kidneys retain plasma proteins by reabsorption of such proteins as they pass through the glomerular filtration barrier. Normal urine protein excretion is up to 150 mg/d. Therefore, the detection of abnormal quantities or types of protein in the urine is considered an early sign of significant renal or systemic disease. When proteinuria occurs, it can cause further renal damage through release of cytokines, inflammation of the renal tubulointerstitium, and progressive fibrosis. Although diabetic nephropathy is a major cause of proteinuria in the United States, proteinuria also occurs in many other disease states that affect protein reabsorption or affect the glomerular barrier (e.g., proliferative glomerulonephritis (e.g., immunoglobulin A nephropathy, membranoproliferative glomerulonephritis, mesangial proliferative glomerulonephritis, anti-GBM disease, renal vasculitis, lupus nephritis, cryoglobulinemia-associated glomerulonephritis, bacterial endocarditis, Henoch-Schönlein purpura, postinfectious glomerulonephritis, hepatitis C), and nonproliferative glomerulonephritis (e.g., membranous glomerulonephritis, minimal-change disease, primary focal segmental glomerulosclerosis (FSGS), fibrillary glomerulonephritis, immunotactoid glomerulonephritis, amyloidosis, hypertensive nephrosclerosis, light-chain disease from multiple myeloma, secondary focal glomerulosclerosis). Additionally, conditions such as obesity and hypertensive nephrosclerosis can cause glomerular hyperfiltration, leading to proteinuria.

Improved pharmacological regimens for the treatment of the proteinuria that occurs in primary renal disease as well as renal disorders secondary to conditions such as diabetes are needed.
BEAUFILS ET AL: "Nephroangiosclerose" EMC - NEPHROLOGIE ELSEVIER, vol. 2, no. 3,1 August 2005, pages 103-124, XP027827841, ISSN: 1638-6248; BIANCHI S ET AL: "Long-term effects if spironolactone on proteinuria and kidney function in patients with chronic kidney disease" KIDNEY INTERNATIONAL, vol. 70, no. 12, December 2006, pages 2116-2123, XP009160945, ISSN: 0085-2538; WO 00/27380 A2 (G.D. SEARLE &CO. ET AL.) 18 May 2000; WO 96/24373 A2 (G:D: SEARLE & CO. ET AL.) 15. August 1996 and RACHMANI, R. ET AL.: "The effect of spironolactone, cilazapril and their combination on albuminuria in patients with hypertension and diabetic nephropathy is independent of blood pressure reduction" DIABETIC MEDICINE, vol. 21, no. 5, 2004, pages 471-475, XP009161130 disclose compositions comprising anti-aldosterone agents such as spironolactone. WO 00/27380 A2 (G.D. SEARLE &CO. ET AL.) 18 May 2000 and WO 96/24373 A2 (G:D: SEARLE & CO. ET AL.) 15. August 1996 disclose compositions comprising spironolactone and an ACE inhibitor.
PUIG ET AL: "Efficacy of indapamide SR Compared With Enalapril in Elderly Hypertensive Patients With Type 2 Diabetes", AMERICAN JOURNAL OF HYPERTENSION, NATURE PUBLISHING GROUP, UNITED STATES, vol. 20, no. 1, 29 December 2006, pages 90-97, XP005818930, ISSN: 0895-7061, DOI: 10.1016/J.AMJHYPER.2006.05.018; GAMBARDELLA S ET AL: "Regression of microalbuminuria in type II diabetic, hypertensive patients after long-term indapamide treatment", AMERICAN HEART JOURNAL, MOSBY-YEAR BOOK INC, US, vol. 122, no. 4, 1 October 1991, pages 1232-1238, XP022951770, ISSN: 0002-8703, DOI: 10.1016/0002-8703(91)90946-F and WO 2008/124611 A1 (DR: REDDY'S LABORATORIES LTD. ET AL.) 16 October 2008 disclose compositions comprising indapamide.
WO 2008/124611 A1 (DR: REDDY'S LABORATORIES LTD. ET AL.) 16 October 2008 discloses compositions comprising indapamide and ramipril.
MOTWANI.J.G: "Combining rennin-angiotension-aldosterone system blockade with diuretic therapy for treatment of hypertension", JOURNAL OF RENIN-ANGIOTENSIN-ALDOSTERONE SYSTEM, vol. 3, 2002, pages 72-78, XP009160965 discloses a therapy for treatment of hypertension.

### SUMMARY OF THE INVENTION

The present invention is defined in the independent claims 1, 2 and 3. Advantageous embodiments are defined in the dependent claims. Some examples described in the present description do not belong to the invention as defined in the claims, but may be useful for the understanding of the invention.

The present invention relates to the use of compositions and to compositions related to the treatment of subject with proteinuria, albuminuria or hyperuricemia. Specifically, the invention relates to the use of an indoline (i.e., indapamide) administered in combination with an anti-aldosterone agent (e.g., spironolactone or eplerenone). The invention finds use in patients suffering from or at risk for proteinuria.

Renoprotection in patients suffering from or at risk for proteinuria is critical to prevent further kidney damage. Similarly, control of hypertension is also needed to prevent further proteinuria and the accompanying kidney damage. However, many of the currently available therapeutic agents for renoprotection and/or treatment of hypertension, whether used alone or in combination, either provide limited renoprotection (especially over long periods of time, such as single ACE inhibitor or ARB agents that become ineffective due to "aldosterone escape"), or carry risk of other dangerous side effects such as electrolyte imbalance. Studies conducted during the course of developing some embodiments of the present invention resulted in the identification of combination therapies that provide unexpectedly synergistic renoprotective (e.g., anti-proteinuria) effects with low risk of undesirable side effects such as electrolyte imbalance or cardiac arrhythmias.

In certain examples of the present disclosure, methods of the present description find use in the treatment of subjects with diabetes or a diabetes-related condition involving e.g., impaired glucose tolerance, impaired insulin sensitivity, impaired insulin production. Such conditions and disease states include but are not limited to diabetes mellitus, type I diabetes, type II diabetes, gestational diabetes, metabolic syndrome, metabolic syndrome X, syndrome X, insulin resistance syndrome, Reaven's syndrome, CHAOS, and malnutrition-related diabetes mellitus. One of skill in the medical arts appreciates that such subjects are at increased risk of kidney disease and proteinuria, and that such subjects find benefit from renoprotective combination therapy, composition, method, and kits which are based on the present invention.

In certain examples of the present disclosure, methods of the present description find use in treatment of conditions or physiological states including but not limited to fluid retention, sodium retention, and hypertension (high blood pressure).

In some examples of the present disclosure, combination therapies, compositions, methods, and kits of the present description find use in treating subjects with diseases or conditions causing or associated with proteinuria, including but not limited to diabetes and diabetes-related diseases (e.g., impaired insulin signaling, diabetes mellitus, type I diabetes, type II diabetes, gestational diabetes, metabolic syndrome, metabolic syndrome X, syndrome X, insulin resistance syndrome, Reaven's syndrome, CHAOS, and malnutrition-related diabetes mellitus), diabetic nephropathy, nephropathy, glomerular disease, liver disease, renal disease, membranous glomerulonephritis, severe cardiac failure, sleep apnea, nephrotic syndrome, drug-induced nephropathy, systemic lupus erythematosus, Goodpasture's syndrome, IgA nephropathy, Alport syndrome, acute post-streptococcal glomerulonephritis (PSGN), bacterial endocarditis, HIV-induced nephropathy, glomerulosclerosis, focal segmental glomerulosclerosis (FSGS), membranous nephropathy (also called membranous glomerulopathy), obesity, and minimal change disease (MCD).

In examples of the present disclosure, methods of the present description comprise testing a subject for proteinuria, followed by administering a combination therapy comprising an indoline (e.g., indapamide) administered in combination with an anti-aldosterone agent (e.g., spironolactone and/or eplerenone). In some examples of the present disclosure, methods of the present invention comprise administering to a subject a combination therapy comprising an indoline (e.g., indapamide) administered in combination with an anti-aldosterone agent (e.g., spironolactone and/or eplerenone), followed by testing the subject for proteinuria, followed by administering a combination therapy comprising an indoline (e.g., indapamide) administered in combination with an anti-aldosterone agent (e.g., spironolactone and/or eplerenone), followed by a second round of testing for proteinuria. In some examples of the present disclosure, methods of the present invention comprise testing a subject for proteinuria, followed by administering a combination therapy comprising an indoline (e.g., indapamide) administered in combination with an anti-aldosterone agent (e.g., spironolactone and/or eplerenone), followed by a second round of testing for proteinuria, and a second administration of a combination therapy comprising an indoline (e.g., indapamide) administered in combination with an anti-aldosterone agent (e.g., spironolactone and/or eplerenone), with this second administration being modified in dose, duration, frequency, or administration route in a manner dependent upon the results of the prior testing.

In some examples of the present disclosure, the description comprises use of a combination therapy comprising an indoline (e.g., indapamide) administered in combination with an anti-aldosterone agent (e.g., spironolactone and/or eplerenone) in the manufacture of a medicament for the treatment of a condition such as to diabetes and diabetes-related diseases (e.g., impaired insulin signaling, diabetes mellitus, type I diabetes, type II diabetes, gestational diabetes, metabolic syndrome, metabolic syndrome X, syndrome X, insulin resistance syndrome, Reaven's syndrome, CHAOS, and malnutrition-related diabetes mellitus), diabetic nephropathy, nephropathy, glomerular disease, liver disease, renal disease, membranous glomerulonephritis, severe cardiac failure, sleep apnea, nephrotic syndrome, drug-induced nephropathy, systemic lupus erythematosus, Goodpasture's syndrome, IgA nephropathy, Alport syndrome, acute post-streptococcal glomerulonephritis (PSGN), bacterial endocarditis, HIV-induced nephropathy, glomerulosclerosis, focal segmental glomerulosclerosis (FSGS), membranous nephropathy (also called membranous glomerulopathy), obesity, and minimal change disease (MCD).

Methods of the present description are not limited by dosing, formulation, administration routes, or temporal administration regimens of the combination therapy comprising an indoline (e.g., indapamide) administered in combination with an anti-aldosterone agent (e.g., spironolactone and/or eplerenone). In some examples of the present disclosure, dosage of the indoline agent (e.g., indapamide) is in the range of about 0.25 mg up to about 2,000 mg per day, with variations necessarily occurring depending on the disease target, the patient, and the route of administration. In some examples of the present disclosure, dosages are administered orally in the range of about 0.05 mg/day to about 20 mg/day, more preferably in the range of about 0.05 mg/day to about 5 mg/day, most preferably in the range of about 0.05 mg/kg to about 0.2 mg per kg of body weight per day. In some examples of the present disclosure, dosage of the anti-aldosterone agent is in the range of about 2 mg up to about 2,000 mg per day, with variations necessarily occurring depending on the disease target, the patient, and the route of administration. In some examples of the present disclosure, dosages are administered orally in the range of about 0.05 mg/day to about 500 mg/day, more preferably in the range of about 0.05 mg/day to about 250 mg/day, most preferably in the range of about 0.05 mg/kg to about 0.2 mg per kg of body weight per day. Administration routes include but are not limited to oral, topical, and parenteral (e.g. intravenous, intramuscular, intraperitoneal, subcutaneous, intrathecal, intraarterial, nasal, or pulmonary administration). In preferred examples of the present disclosure, administration is oral. The description is not limited by temporal aspects of administration of a combination therapy comprising an indoline (e.g., indapamide) administered in combination with an anti-aldosterone agent (e.g., spironolactone and/or eplerenone). The indoline agent (e.g., indapamide) and anti-aldosterone agent (e.g., spironolactone and/or eplerenone) may be administered simultaneously or sequentially without regard to order of administration. The combination therapy may be administered between 1 and 50 times per day, or less often (e.g., every other day, every three days, every 4 days, every 5 days, every 6 days, once a week, once a month, once every three months.) The combination therapy may be continued for 1 day, 1 week, 1 month, 1 year, 10 years, or more than 10 years. In some examples of the present disclosure, the agents are administered in combination using multiple delivery vehicles each of a single type (e.g., more than one type of delivery vehicle (e.g., pill, capsule, tablet) each with a single compound per delivery vehicle). In some examples of the present disclosure, the agents are administered in combination using a single delivery vehicle (e.g., pill, capsule, tablet) comprising more than one agent (e.g., an indoline agent (e.g., indapamide) and an anti-aldosterone agent in a single delivery vehicle (e.g., pill, capsule, tablet). In some examples of the present disclosure, the agents are administered in combination in single delivery vehicle (e.g., pill, tablet, capsule) in further combination with other therapeutic agents (e.g., other active anti-hypertensive agents (e.g., one or more beta-blockers).

In some examples of the present disclosure, combination therapies, compositions, methods, and kits of the present description find use in patients presenting with proteinuria and/or hypertension wherein proteinuria and/or hypertension remains uncontrolled despite prior or continued treatment with one or more other therapies (e.g., treatment with agents other than an indoline (e..g, indapamide) and an anti-aldosterone agent (e.g., spironolactone and/or eplerenone)). In some examples of the present disclosure, combination therapies, compositions, methods, and kits of the present invention find use in subpopulations of patients (e.g., ethnic minority and majority populations (e.g., African-American, Hispanic, Asian, Native American, multi-ethnic minority populations, populations distinguished by geographic ancestry, populations distinguished by culture, diet, climate, environmental exposures, etc)). In some examples of the present disclosure, subpopulations of patients for which combination therapies, compositions, methods, and kits of the present invention find use are identified on the basis of common biomarkers indicating shared geographical ancestry.

The present disclosure provides a composition comprising an indoline, which is selected from indapamide and an anti-aldosterone agent, selected from spironolactone or eplerenone. In some examples of the present disclosure, the indoline and the anti-aldosterone agent are formulated for simultaneous administration in a single delivery vehicle. In some examples of the present disclosure, the single delivery vehicle is formulated for oral administration.

The present description provides a composition for use in a method for treating or preventing a condition such as proteinuria, albuminuria, hyperuricemia, in a subject comprising administering a combination therapy comprising an indoline and an anti-aldosterone agent. In some examples of the present disclosure, the indoline and the anti-aldosterone agent are administered as separate compositions. In some examples of the present disclosure, the indoline and the anti-aldosterone agent are administered as a single composition. In some examples of the present disclosure, the indoline is indapamide. In some examples of the present disclosure, the anti-aldosterone agent is an agent such as an aldosterone receptor blocker or an inhibitor of aldosterone synthesis. In some examples of the present disclosure, the aldosterone receptor blocker is an agent such as spironolactone or eplerenone. In some examples of the present disclosure, the indoline and the anti-aldosterone agent are formulated for simultaneous administration in a single delivery agent.

In certain examples of the present disclosure, the present description provides a method of treating a subject with a disease such as impaired insulin signaling, diabetes mellitus, type I diabetes, type II diabetes, gestational diabetes, metabolic syndrome, metabolic syndrome X, syndrome X, insulin resistance syndrome, Reaven's syndrome, CHAOS, malnutrition-related diabetes mellitus, diabetic nephropathy, nephropathy, glomerular disease, liver disease, renal disease, membranous glomerulonephritis, severe cardiac failure, nephrotic syndrome, systemic lupus erythematosus, Goodpasture's syndrome, IgA nephropathy, Alport syndrome, acute post-streptococcal glomerulonephritis (PSGN), bacterial endocarditis, HIV-induced nephropathy, glomerulosclerosis, focal segmental glomerulosclerosis (FSGS), membranous nephropathy (membranous glomerulopathy), obesity, minimal change disease (MCD), heart failure, hypertension, liver failure, acute renal failure, chronic kidney disease, congestive heart failure, proteinuric kidney disease, diabetic nephropathy, glomerulonephritis, amyloidosis, and/or drug-induced renal disease comprising administering a composition comprising an indoline and an anti-aldosterone agent. In some examples of the present disclosure, the indoline is indapamide. In some examples of the present disclosure, the anti-aldosterone agent is an agent such as an aldosterone receptor blocker or an inhibitor of aldosterone synthesis. In some examples of the present disclosure, the aldosterone receptor blocker is an agent such as spironolactone or eplerenone. In some examples of the present disclosure, the indoline and the anti-aldosterone agent are formulated for simultaneous administration in a single delivery agent.

In certain examples of the present disclosure, the present description provides a method of promoting a state in a subject such as homeostasis of serum potassium level, homeostasis of serum uric acid level, normal cardiac ventricular rhythm, and/or normal QT interval comprising administering a composition comprising an indoline and an anti-aldosterone agent. In some examples of the present disclosure, the indoline is indapamide and the anti-aldosterone agent is an agent such as an aldosterone receptor blocker or an inhibitor of aldosterone synthesis. In some examples of the present disclosure, the method further comprises administration of an agent having a function such as preventing or reducing proteinuria, promoting natriuresis, and/or preventing or reducing hypertension. In some examples of the present disclosure, the agent is an agent such as an angiotensin converting enzyme inhibitor, an anti-renin agent, an endothelin antagonist, a calcium channel blocker, a beta blocker and/or an anti-inflammatory agent.

In certain examples of the present disclosure, the present description provides use of a combination therapy comprising an indoline and an anti-aldosterone agent for the treatment of a condition such as proteinuria, albuminuria, sodium retention, hypokalemia, hyperkalemia, hyperuricemia, dyslipidemia, or hyperglycemia in a subject.

In certain examples of the present disclosure, the present description provides use of a combination therapy comprising an indoline and an anti-aldosterone agent for the treatment or prevention of a condition such as proteinuria, albuminuria, sodium retention, hypokalemia, hyperkalemia, hyperuricemia, dyslipidemia, or hyperglycemia in a subject comprising providing a subject suffering from or at risk for a condition such as proteinuria, albuminuria, sodium retention, hypokalemia, hyperkalemia, hyperuricemia, dyslipidemia, or hyperglycemia; providing the combination therapy comprising an indoline and an anti-aldosterone agent; and measuring a benefit related to a condition such as proteinuria, albuminuria, sodium retention, hypokalemia, hyperkalemia, hyperuricemia, dyslipidemia, or hyperglycemia.

In certain examples of the present disclosure, the present description provides use of a combination therapy comprising an indoline and an anti-aldosterone agent for the treatment of a subject with a disease such as impaired insulin signaling, diabetes mellitus, type I diabetes, type II diabetes, gestational diabetes, metabolic syndrome, metabolic syndrome X, syndrome X, insulin resistance syndrome, Reaven's syndrome, CHAOS, malnutrition-related diabetes mellitus, diabetic nephropathy, nephropathy, glomerular disease, liver disease, renal disease, membranous glomerulonephritis, severe cardiac failure, nephrotic syndrome, systemic lupus erythematosus, Goodpasture's syndrome, IgA nephropathy, Alport syndrome, acute post-streptococcal glomerulonephritis (PSGN), bacterial endocarditis, HIV-induced nephropathy, glomerulosclerosis, focal segmental glomerulosclerosis (FSGS), membranous nephropathy (membranous glomerulopathy), obesity, minimal change disease (MCD), heart failure, hypertension, liver failure, acute renal failure, chronic kidney disease, congestive heart failure, proteinuric kidney disease, diabetic nephropathy, glomerulonephritis, amyloidosis, or drug-induced renal disease comprising administering said combination therapy comprising an indoline and an anti-aldosterone agent.

In certain examples of the present disclosure, the present description provides use of a combination therapy comprising an indoline and an anti-aldosterone agent for the promotion of a state in a subject such as homeostasis of serum potassium level, homeostasis of serum uric acid level, normal cardiac ventricular rhythm, or normal QT interval comprising administering said composition comprising an indoline and an anti-aldosterone agent.

In certain examples of the present disclosure, the present description provides a composition comprising an indoline and an anti-aldosterone agent.

Additional examples of the present disclosure will be apparent to persons skilled in the relevant art based on the teachings contained herein.

### DESCRIPTION OF THE DRAWING

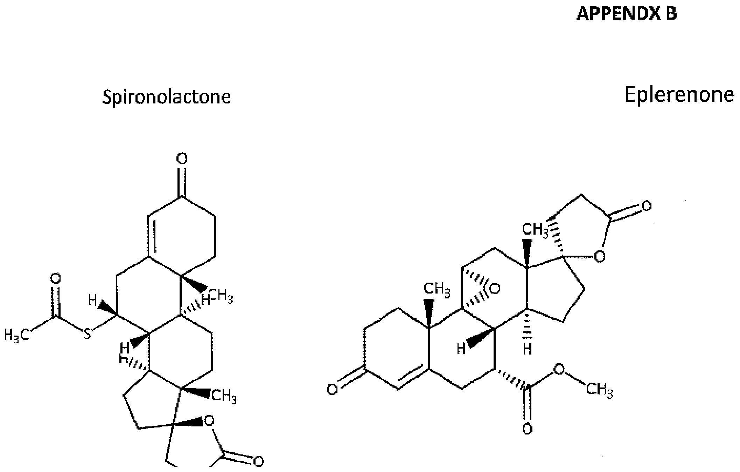

Figure 1 shows a flowchart for methodology for a study comparing, e.g., a combination therapy of indapamide and spironolactone versus thiazide diuretic for additive control of albuminuria in diabetic nephropathy.

### DEFINITIONS

To facilitate an understanding of the present description, a number of terms and phrases are defined below:
As used herein, the term "subject" refers to any animal (*e.g.,* a mammal), including, but not limited to, humans, non-human primates, rodents, and the like, which is to be the recipient of a particular treatment. Typically, the terms "subject" and "patient" are used interchangeably herein in reference to a human subject.

As used herein, the term "proteinuria" refers to a condition in which excess protein is present in the urine of a subject. In human subjects, proteinuria is often diagnosed by urinanlysis. Clinically, proteinuria may be determined when there is presence of urinary protein in amounts exceeding 0.3 g in a 24-hour urine collection or in concentrations more than 1 g per liter (1+ to 2+ by standard turbidometric methods) in a random urine collection on two or more occasions at least 6 hours apart. "Proteinuria" is sometimes considered to be synonymous with "albuminuria", although the latter refers specifically to presence of albumin protein in the urine, whereas proteinuria may encompass the presence of proteins other than albumin.

As used herein, the term "subject suspected of having diabetes" refers to a subject that presents one or more symptoms indicative of diabetes or a diabetes-related condition (e.g., diabetes mellitus type 1, diabetes mellitus type 2, gestational diabetes, pre-diabetes, metabolic syndrome, syndrome X) (*e.g.,* polyuria, polydipsia, nocturia, fatigue, weight loss) or is being screened for diabetes (*e.g*., during a routine physical). A subject suspected of having diabetes or a diabetes-related condition may also have one or more risk factors. A subject suspected of having diabetes or a diabetes-related condition has generally not been tested for diabetes or a diabetes-related condition. However, a "subject suspected of having diabetes" encompasses an individual who has received a preliminary diagnosis (*e.g*., a random plasma glucose level) but for whom a confirmatory test (*e.g*., fasting glucose plasma level, hemoglobin HA1C, glucose tolerance test) has not been done or for whom the type of diabetes is not known. A "subject suspected of having diabetes" is sometimes diagnosed with diabetes and is sometimes found to not have diabetes.

As used herein, the term "subject diagnosed with diabetes" refers to a subject who has been tested and found to have diabetes or a diabetes-related condition (e.g., diabetes mellitus type 1, diabetes mellitus type 2, gestational diabetes, pre-diabetes, metabolic syndrome, syndrome X) (e.g., a random plasma glucose level of ≥200 mg/dL or greater, a fasting glucose plasma level of ≥110 mg/dL occurring on two separate occasions, or 2 hours post glucose load (75 g) plasma glucose of ≥200 mg/dL on two separate occasions). Diabetes may be diagnosed using any suitable method, including but not limited to, measurements of random plasma glucose level, fasting plasma glucose level, hemoglobin A1c (HbA_{1c}) levels, glycosylated hemoglobin (GHb) levels. A "preliminary diagnosis" is one based only on presenting symptoms (*e.g*., polyuria, polydipsia, nocturia, fatigue, weight loss).

As used herein, the term "initial diagnosis" refers to a test result of initial diabetes diagnosis that reveals elevated plasma glucose levels (*e.g*., random plasma glucose levels).

As used herein, the term "subject at risk for diabetes" refers to a subject with one or more risk factors for developing diabetes or a diabetes-related condition. Risk factors include, but are not limited to, obesity (particularly central or abdominal obesity), ethnicity, gender, age, genetic predisposition, diet, lifestyle (particularly sedentary lifestyle), and diseases or conditions that can lead to secondary diabetes (e.g., treatment with glucocorticoids, Cushing syndrome, acromegaly, pheochromocytoma, surgery, other endocrine disorders).

As used herein, the term "characterizing diabetes in subject" refers to the identification of one or more properties of diabetes disease or a diabetes-related disease in a subject, including but not limited to, plasma glucose levels (random, fasting, or upon glucose challenge); HbA_{1c} levels; glycosylated hemoglobin (GHb) levels; microalbumin levels or albumin-to-creatinine ratio (ACR); insulin levels; C-peptide levels; antibodies to insulin, islet cells, or glutamic acid decarboxylase (GAD); levels of anti-GAD65 antibody (e.g., as an indicator of latent autoimmune diabetes of adults).

As used herein, the term "providing a prognosis" refers to providing information regarding the impact of the presence of diabetes on a subject's future health (e.g., expected morbidity or mortality, the likelihood of getting diabetes, and the risk of diabetes-related complications).

As used herein, the term "non-human animals" refers to all non-human animals including, but not limited to, vertebrates such as rodents, non-human primates, ovines, bovines, ruminants, lagomorphs, porcines, caprines, equines, canines, felines, aves, etc.

As used herein, the term "eukaryote" refers to organisms distinguishable from "prokaryotes." It is intended that the term encompass all organisms with cells that exhibit the usual characteristics of eukaryotes, such as the presence of a true nucleus bounded by a nuclear membrane, within which lie the chromosomes, the presence of membrane-bound organelles, and other characteristics commonly observed in eukaryotic organisms. Thus, the term includes but is not limited to such organisms as fungi, protozoa, and animals (*e.g.,* humans).

As used herein, the term "*in vitro"* refers to an artificial environment and to processes or reactions that occur within an artificial environment. In vitro environments can consist of, but are not limited to, test tubes and cell culture. The term *"in vivo"* refers to the natural environment (*e.g.,* an animal or a cell) and to processes or reaction that occur within a natural environment.

As used herein, the term "sample" is used in its broadest sense. In one sense, it is meant to include a specimen or culture obtained from any source, as well as any biological. Biological samples may be obtained from animals (including humans) and encompass fluids, solids, tissues, and gases. Biological samples include blood products, such as plasma, serum and the like. Such examples are not however to be construed as limiting the sample types applicable to the present invention.

As used herein, the term "effective amount" refers to the amount of a compound (*e.g.,* a combination of an indoline and an anti-aldosterone compound as described herein) sufficient to effect beneficial or desired results. An effective amount can be administered in one or more administrations, applications or dosages and is not limited to or intended to be limited to a particular formulation or administration route.

As used herein, the term "co-administration" refers to the administration of at least two agent(s) (*e.g.,* an indoline and an anti-aldosterone as described herein) or therapies to a subject. In some examples of the present disclosure, the co-administration of two or more agents/therapies is concurrent. In other examples of the present disclosure, a first agent/therapy is administered prior to a second agent/therapy. Those of skill in the art understand that the formulations and/or routes of administration of the various agents/therapies used may vary. The appropriate dosage for co-administration can be readily determined by one skilled in the art. In some examples of the present disclosure, when agents/therapies are co-administered, the respective agents/therapies are administered at lower dosages than appropriate for their administration alone. Thus, co-administration is especially desirable in examples of the present disclosure where the co-administration of the agents/therapies lowers the requisite dosage of a known potentially harmful (*e.g.,* toxic) agent(s).

As used herein, the term "pharmaceutical composition" refers to the combination of an active agent with a carrier, inert or active, making the composition especially suitable for diagnostic or therapeutic use *in vitro, in vivo* or *ex vivo.*

As used herein, the term "pharmaceutically acceptable carrier" refers to any of the standard pharmaceutical carriers, such as a phosphate buffered saline solution, water, emulsions (*e.g.,* such as an oil/water or water/oil emulsions), and various types of wetting agents. The compositions also can include stabilizers and preservatives. For examples of carriers, stabilizers and adjuvants, see *e.g.,* Martin, Remington's Pharmaceutical Sciences, 15th Ed., Mack Publ. Co., Easton, PA (1975).

As used herein, the term "pharmaceutically acceptable salt" refers to any pharmaceutically acceptable salt (*e.g.,* acid or base) of a compound of the present invention which, upon administration to a subject, is capable of providing a compound of this invention or an active metabolite or residue thereof. As is known to those of skill in the art, "salts" of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Examples of bases include, but are not limited to, alkali metals (*e.g.,* sodium) hydroxides, alkaline earth metals (*e.g.,* magnesium), hydroxides, ammonia, and compounds of formula NW₄⁺, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

For therapeutic use, salts of the compounds of the present description are contemplated as being pharmaceutically acceptable. However, salts of acids and bases that are non-pharmaceutically acceptable may also find use, for example, in the preparation or purification of a pharmaceutically acceptable compound.

As used herein, the term "instructions for administering said compound to a subject," and grammatical equivalents thereof, includes instructions for using the compositions contained in a kit for the treatment of proteinuria and/or hypertension (*e.g.,* providing dosing, route of administration, decision trees for treating physicians for correlating patient-specific characteristics with therapeutic courses of action). The indoline and/or anti-aldosterone combination therapies of the present description (e.g. as presented herein) can be packaged into a kit, which may include instructions for administering the compounds to a subject.

As used herein, the term "obesity" refers to the state of being in excess of one's ideal weight, wherein determination of ideal weight takes into account multiple factors including but not limited to one's height, age, sex, and build. In some examples of the present disclosure, "obese" is defined as having a body mass index (BMI) of 30 or greater.

As used herein, the term "body mass index" or "BMI" is defined as a person's weight in kilograms (kg) divided by their height in meters (m) squared. While BMI strongly correlates with overall fat content, one of ordinary skill in the art understands that some very muscular people may have a high BMI without undue health risks.

As used herein, the term "diabetes" or "diabetes-related condition" refers to a metabolic or endocrinological disease, condition, or state affecting control of glycemia. Diabetes or diabetes-related conditions may affect sensitivity to insulin or production of insulin. Examples of diabetes or diabetes-related diseases or condition include but are not limited to impaired insulin signaling, diabetes mellitus, type I diabetes, type II diabetes, gestational diabetes, metabolic syndrome, metabolic syndrome X, syndrome X, insulin resistance syndrome, Reaven's syndrome, CHAOS, and malnutrition-related diabetes mellitus.

As used herein, the term "edema" refers to an abnormal accumulation of fluid beneath the skin, or in one or more cavities of the body. Causes of edema which are generalized to the whole body can cause edema in multiple organs and peripherally. For example, severe heart failure (e.g., congestive heart failure) can cause pulmonary edema, pleural effusions, ascites and peripheral edema, the last of which effects can also derive from less serious causes. Organ-specific edema types include cerebral edema, pulmonary edema, pleural efflusions, corneal edema, periorbital edema, cutaneous edema, myxedema, edema of the feet, and lymphedema.

As used herein, the term "congestive heart failure" refers to an imbalance in pump function in which the heart fails to adequately maintain the circulation of blood. In the New York Heart Association's functional classification of CHF, Class I describes a patient whose normal physical activity is not limited by symptoms. Class II occurs when ordinary physical activity results in fatigue, dyspnea, or other symptoms. Class III is characterized by a marked limitation in normal physical activity. Class IV is defined by symptoms at rest or with any physical activity.

As used herein, the term "indoline" refers to compounds comprising an indoline moiety, particularly those having diuretic and/or anti-hypertensive function. Examples of indulines include but are not limited to 1-[(tert-butylimino)methyl]-2-(3-indolyl) indoline HCl and derivatives thereof; 1-hydroxyalkanamine pyrano(3,4-B) indole derivatives (see, e.g., PCT App. No. PCT/US1979/000256; herein incorporated by reference in its entirety); and 3-sulphamoyl-4-chlorobenzamides in the indoline and iso-indoline series (e.g., indapamide [4-chloro-N-(2-methyl-2,3-dihydroindol-1-yl)-3-sulfamoyl-benzamide]). In some examples of the present disclosure, indoline compounds are described as compounds of formula I 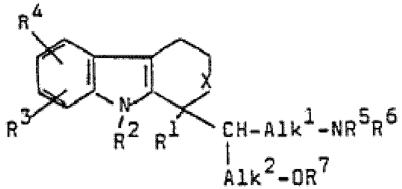 in which R1 is lower alkyl; R2 is hydrogen or lower alkyl; R3 and R4 each independently is hydrogen, lower alkyl halo, nitro, trifluoromethyl or lower alkoxy; R5 and R6 each independently is hydrogen or lower alkyl or R5 and R6 together with the nitrogen atom form a pyrrol-1-yl, piperidino or morpholino ring; R7 is hydrogen or lower alkyl; Alk1 and Alk2 each independently is a straight or branched chain lower alkylene having one to six carbon atoms; and X is oxa or thia, and therapeutically acceptable acid addition salts thereof. The above compounds are diuretic agents, which also possess anti-hypertensive activity. They may be used alone or with other diuretic agents. Pharmaceutical compositions containing the compounds and processes for preparing the compounds are also described.

As used herein, the term "anti-aldosterone agent" or "aldosterone antagonist" refers to an agent that antagonizes an effect or function (e.g., a physiological effect or function, a biochemical effect or function, a molecular effect or function) of aldosterone. Anti-aldosterone agents alsoinclude but are not limited to spironolatone, eplerenone, canrenone, anti-aldosterone antibodies, potassium canrenoate, carbolactones such as spirorenone, progestin analogs such as drospirenone, mesperinone and combined B-Type Natriuretic Peptide and Vasopressin-2 Receptor antagonism with Tolvaptan.

The present description relates to methods, compositions, and kits for the treatment of subjects at risk for or suffering from proteinuria and hypertension (e.g., proteinuria and/or hypertension arising from primary renal disease (e.g., focal segmental glomerulosclerosis, glomerular disease) or secondary to other conditions (e.g., diabetes, diabetic nepropathy, cirrhosis, liver disease). Specifically, the description relates to methods involving combination therapy wherein an indoline (e.g., indapamide) is administered in combination with an anti-aldosterone agent (e.g., spironolactone and/or eplerenone).

Proteinuria is caused by many diseases and conditions, as detailed below. Urinary protein loss is a frequent hallmark of the development and progression of diabetic nephropathy, a major source of morbidity for diabetic patients. Optimal control of blood pressure retards the progression of diabetic nephropathy, but in most cases, requires multiple medications that provide additive benefits via different mechanisms. Investigations conducted during the course of developing some examples of the present disclosure of the present description identified combination therapies providing renoprotection, e.g., by preventing or treating proteinuria. In some examples of the present disclosure, combination therapies comprising an indoline agent (e.g., indapamide) plus an anti-aldosterone agent (e.g., spironolactone, eplerenone) provide superior lowering of proteinuria (e.g., albuminuria) in comparison to, e.g., traditional thiazide diuretics. While the present invention is not limited to any particular mechanism, and an understanding of the mechanism is not necessary to practice the present invention, it is contemplated that such renoprotective effects occur independently of blood pressure changes, either when combination therapy examples of the present disclosure of the present invention are administered alone or when they are added to other therapies (e.g., added to first-line drugs that block the renin-angiotensin-aldosterone system).

Combination therapy, composition, and method examples of the present disclosure of the present description provide numerous unexpected benefits over existing therapies and over the administration of single agents (e.g., a single indoline agent, a single anti-aldosterone agent). In some examples of the present disclosure, combination therapies of the present description markedly reduce proteinuria. In some examples of the present disclosure, combination therapies of the present description provide synergistic effects in proteinuria reduction as compared to administration of either agent alone (e.g., a single indoline agent, a single anti-aldosterone agent). In some examples of the present disclosure, combination therapies of the present description provide increased reduction in proteinuria in comparison to other combinations (e.g., an angiotensin-converting enzyme inhibitor (ACE inhibitor) in combination with an angiotensin receptor blocker (ARB); an ACE inhibitor and a diuretic; an ARB and a diuretic; an ACE inhibitor and an anti-aldosterone agent (e.g., spironolactone); an ARB and an anti-aldosterone agent (e.g., spironolactone); an ACE inhibitor and a calcium channel blocker; an ACE inhibitor and a beta blocker; an ARB and a beta blocker). In some examples of the present disclosure, combination therapies of the present description result in fewer undesirable side effects as compared to side effects occurring when either single agent is administered alone (e.g., a single indoline agent, a single anti-aldosterone agent), such side effects including but not limited to effects on cardiac ion channels, effects on serum potassium levels, effects on serum magnesium levels, effects on serum uric acid levels.

The deleterious effects of elevated levels of aldosterone on the heart and circulatory system have resulted in the use of ACE inhibitor agents and ARB agents in an attempt to inhibit the renin-angiotensin-aldosterone system. However, experimental evidence and clinical observations have shown that ACE inhibition only reduces aldosterone levels transiently. This so-called "aldosterone escape" phenomenon occurs despite treatment with ACE inhibitors and ARBs, alone or in combination, irrespective of doses used. The mechanism of aldosterone escape is unknown, but could be related to inadequate ACE inhibition and the existence of multiple pathways for aldosterone release. A similar phenomenon occurs during the inhibition of angiotensin II production by ACE inhibitors and has been called "angiotensin reactivation". Both of these phenomena have been associated with poor patient prognosis. Notably, aldosterone escape has been reported to occur more frequently than angiotensin reactivation. In some examples of the present disclosure, combination therapies of the present description provide inhibition of the renin-angiotensin-aldosterone system without triggering aldosterone escape or angiotensin reactivation.

Combination therapy, composition, and method examples of the present disclosure of the present description further provide benefits for reducing the risk of drug-induced cardiac arrhythmia (e.g., Torsade de Pointes, as described below). When used as a single agent, indapamide causes an increased incidence in a form of tachyarrhythmia associated with long QT interval known as Torsade de Pointes (TdP). While the present description is not limited to any particular mechanism, and an understanding of the mechanism is not necessary to practice the present description, it is contemplated that aldosterone antagonists accelerate repolarization, shorten QT interval and thereby reduce the risk of TdP by major effects on inhibition of inward Na⁺ and Ca⁺² currents. Therefore, during the course of developing some examples of the present disclosure of the present description, it was contemplated that combining antialdosterone agents spironolactone and/or eplerenone with indapamide helped to mitigate the risk of TdP from use of indapamide. Furthermore, hypokalemia and hypomagnesemia slow ventricular repolarization, prolong QT and thereby predispose to TdP. These electrolyte disturbances induced by indapamide were further mitigated by combination with anti-aldosterone agents.

### A. Proteinuria

Proteinuria refers to the presence of an excess of serum proteins in the urine. There are three main mechanisms thought to cause proteinuria: 1) glomerular disease; 2) overflow proteinuria due to increased quantity of proteins in serum; and 3) low reabsorbtion at proximal tubule (fanconi). Proteinuria is often diagnosed by a simple dipstick test, although if urine is dilute, it is possible for the test to give a false negative even with nephrotic range proteinuria. False negatives may also occur if the protein in the urine is composed mainly of globulins or Bence-Jones Proteins because the reagent on the test strips, Bromphenol blue, is highly specific for albumin. Traditionally, dipstick protein tests are quantified by measuring the total quantity of protein in a 24-hour urine collection test, and abnormal globulins by specific requests for protein electrophoresis. Alternatively, the concentration of protein in the urine may be compared to the creatinine level in a spot urine sample. This is termed Protein/Creatinine Ratio (PCR). Proteinuria may be defined as a Protein:creatinine ratio >45 mg/mmol (which is equivalent to Albumin:creatinine ratio of >30 mg/mmol) with very high levels of nephrotic syndrome being for PCR > 100 mg/mmol.

Proteinuria may be a sign of renal (kidney) damage. Since serum proteins are readily reabsorbed from urine, the presence of excess protein indicates either an insufficiency of absorption or impaired filtration. Diabetics may suffer from damaged nephrons and develop proteinuria. The most common cause of proteinuria in the United States is diabetes, and in any person with proteinuria and diabetes the etiology of the underlying proteinuria should be separated into two categories: diabetic proteinuria versus a category other than diabetic proteinuria.

With severe proteinuria, general hypoproteinemia can develop, which results in diminished oncotic pressure. Symptoms of diminished oncotic pressure may include ascites, edema, and hydrothorax.

Proteinuria of glomerular origin is a manifestation of generalized vascular disease and is therefore associated with adverse cardiovascular outcomes. Experimental and clinical evidence supports the association between severity of proteinuria and accelerated progression of chronic kidney disease and resulting renal failure. Leakage of protein across glomerular capillary basement membrane into the urinary space is followed by uptake of protein by the renal tubular epithelial cell, release of cytokines such as monocyte chemoattractant protein-1 and RANTES, resultant inflammation in renal tubulointerstitium, progressive fibrosis and thereby accelerated progression of renal disease. Furthermore, recent evidence suggests that complement anaphylotoxin C3a is an important mediator of proteniuric glomerular and tubulointerstitial injury and can induce transition of renal tubular epithelial cells from an epithelial phenotype to phenotypic and functional characteristics of mesenchymal cells.

Proteinuria may be a feature of conditions including but not limited to: nephrotic syndromes (i.e. intrinsic renal failure); pre-eclampsia; eclampsia; toxic kidney lesions; collagen vascular diseases (e.g. systemic lupus erythematosus); dehydration; glomerular diseases such as membranous glomerulonephritis, focal segmental glomerulonephritis, and minimal change disease (lipoid nephrosis); strenuous exercise; stress; benign orthostatic (postural) proteinuria; focal segmental glomerulosclerosis (FSGS); IgA nephropathy (i.e. Berger's disease); IgM nephropathy; membranoproliferative glomerulonephritis; membranous nephropathy; sarcoidosis; Alport's syndrome; diabetes mellitus; drugs (e.g. NSAIDs, nicotine, penicillamine, gold and other heavy metals, ACE inhibitors, antibiotics, opiates especially heroin); Fabry's disease; infections (e.g. HIV, syphilis, hepatitis, post-streptococcal infection); aminoaciduria; Fanconi syndrome,;hypertensive nephrosclerosis; interstitial nephritis; sickle cell disease; hemoglobinuria; multiple myeloma; organ rejection; Ebola hemorrhagic fever; Nail Patella Syndrome; Familial Mediterranean fever; HELLP Syndrome; Wegener's Granulomatosis; and Glycogen Storage Disease Type 1.

### B. Indapamide

Indapamide is a diuretic with a unique structure that is distinct from diuretics belonging to the class of thiazides, loop diuretics, carbonic anhydrase inhibitors and potassium sparing diuretics such as triamterene, amiloride or aldosterone receptor antagonists such as spironolactone and eplerenone. The US trade name for indapamide is Lozol. Indapamide is marketed as Natrilix outside of the US. Indapamide is in a class of antihypertensive/diuretics, the indolines. Its molecule contains both a polar sulfamoyl chlorobenzamide moiety and a lipid-soluble methylindoline moiety. It differs chemically from the thiazides in that it does not possess the thiazide ring system and contains only one sulfonamide group. The chemical name of indapamide is 4-Chloro-N- (2-methyl-lindolinyl) -3-Sulfamoylbenzamide.

Indapamide resembles thiazide diuretics in renal actions, in that both agents inhibit sodium and chloride reabsorption in the distal tubule. Consequently, indapamide exhibits diuretic properties similar to thiazide diuretics. Furthermore, like thiazides, indapamide administration is associated with hypokalemia, hyponatremia, hypercalcemia and hyperuricemia. However, the distinct and unique structure of indapamide confers marked differences in extra-renal actions, compared with thiazides, such effects including but not limited to: calcium channel antagonistic activity; blockage of the tissue rennin-angiotensin system; inhibition of the adrenerginc system thereby lowering plasma catchecholamine levels; and improvement in vascular reactivity by enhancement of endothelium-dependent vascular relaxation (in contrast to thiazides, which impair vascular reactivity by inhibition of endothelium-dependent vascular relaxation). Therefore, the use of indapamide confers several advantages over thiazide diuretics, such advantages including but not limited to antihypertensive action manifest in the absence of renal function, as in anuric patients on dialysis; a lack of undesirable decrease in glomerular filtration rate (GFR) and renal plasma flow (in contrast to thiazide durietics); reduction of proteinuria effectively in proteinuric states independent of anti-hypertensive action; and absence of increased risk of metabolic syndrome (Ames et al. (1996) Am. J. Cardiol. 77:12B-16B; herein incorporated by reference in its entirety) in contrast to thiazides, which can increase risk of metabolic syndrome; and utility even when GFR is lower than 30 ml/min.

### C. Anti-aldosterone Agents

The deleterious effects of elevated levels of aldosterone on the cardiovascular system have been described (Hollenberg (2004) Kidney Int. 66:1-9, herein incorporated by reference in its entirety), and it was widely assumed that inhibition of the renin-angiotensin aldosterone system (RAAS) with ACEI or ARB agents would impair aldosterone release sufficiently, since aldosterone is a downstream target of this pathway. However, RAAS inhibition only reduces aldosterone levels transiently, because of an "aldosterone escape" phenomenon that occurs irrespective of the doses used (Sato et al. (2003) Am. J. Hypertens. 16:781-788; herein incorporated by reference in its entirety), which may affect as many as 40% of all type 2 diabetic patients with nephropathy (Sato et al. (2003) Hypertension 41:64-68; herein incorporated by reference in its entirety). The mechanism may be related to inadequate ACE inhibition and the existence of multiple pathways leading to aldosterone release. Schjoedt *et al.* demonstrated in type 1 diabetic patients with nephropathy that losartan 100 mg daily suppressed aldosterone only for the first few months in about one-third of subjects, followed by a return of plasma aldosterone to baseline values (Schjoedt et al. (2004) Diabetologia 47:1936-1939; herein incorporated by reference in its entirety). Aldosterone escape in these patients was associated with doubling of the rate of decline in GFR compared to the two-thirds of patients who did not experience aldosterone escape over a period of about 9 months (Schjoedt et al. (2004) Diabetologia 47:1936-1939; herein incorporated by reference in its entirety). Yoneda *et al.* observed a similar "escape" with valsartan and candesartan, affecting some 22% of their diabetic subjects (Yoneda et al. (2007) Am. J. Hyperten. 20:1329-1333; herein incorporated by reference in its entirety). Thus, it is clear that RAAS blockade with these agents alone is insufficient to eliminate the adverse effects of aldosterone.

Spironolactone is a competitive antagonist of the aldosterone-sensitive sodium channel in the distal cortical tubule (Horisberger et al. (1987) Ren. Physiol. 10:198-220; herein incorporated by reference in its entirety). Because of its natriuretic properties, spironolactone is an effective anti-hypertensive agent that provides additive effects when used in combination with other anti-hypertensive agents, including ACEI or ARB agents. Spironolactone is uniquely effective for offsetting the hypokalemia typically associated with other diuretics, and may be particularly efficacious for states of hyperaldosteronism (Lim et al. (2001) J. Hypertens. 19:353-361; herein incorporated by reference in its entirety), or edematous states like ascites where a more gentle diuresis is warranted (Gines et al. (1992) Drugs 43:316-332; herein incorporated by reference in its entirety). Spironolactone has also been shown to provide substantial survival benefit in patients with advanced congestive heart failure (CHF), as demonstrated in the landmark RALES trial (Pitt et al. (1999) New Engl. J. Med. 341:709-717; herein incorporated by reference in its entirety). Side effects, including hyperkalemia or anti-androgen effects may be troubling for a subset of susceptible patients, but in most cases, the benefits still outweigh these risks (Sica (2007) Curr. Drug Saf. 2:71-77; herein incorporated by reference in its entirety). Effects on serum calcium and uric acid levels appear to be negligible (Garca et al. (1991) J. Clin. Pharmacol. 31:455-461; herein incorporated by reference in its entirety). In addition, given the deleterious effects of aldosterone on the vasculature, and the role of inflammation and fibrosis on the pathogenesis of aldosterone-induced vascular damage leading to renal parenchymal damage, spironolactone may also possess anti-inflammatory and anti-fibrotic properties, as was previously shown in animal models (Han et al. (2006) 70:111-120; herein incorporated by reference in its entirety).

Addition of spironolactone to diabetic nephropathy patients with "aldosterone escape" despite ACEIs improves albuminuria independent of further BP lowering (Sato et al., (2003) Hypertension 41:64-68; herein incorporated by reference in its entirety). Similar benefits have also been demonstrated in non-diabetic patients with substantial macroalbuminuria (Sato et al. 2005) Am. J. Hypertens. 18:44-49; herein incorporated by reference in its entirety) and diabetic patients with nephrotic range proteinuria ((Schjoedt et al. (2006) Kidney Int. 70:536-542; herein incorporated by reference in its entirety). In the study by Rachmani *et al.* comparing spironolactone to cilazapril or their combination, the albuminuria lowering effect of spironolactone was statistically greater than that of the ACEI, and was additive to the ACEI when used together (Rachmani et al. (20040 Diabet. Med. 21:471-475; herein incorporated by reference in its entirety). Spironolactone has additive benefits for reducing albuminuria in diabetic patients previously treated with either ACEIs or ARBs, although worsening hyperkalemia and reductions in GFR may be potential adverse effects (ven den Meiracker et al. (2006) J. Hypertens. 24:2285-2292; Saklayen et al. (2008) J. Investig. Med. 56:714-719; Davidson et al. (2008) Endocr. Pract. 14:985-992; Preston et al. (2009) Hypertens. 53:754-760; each herein incorporated by reference in its entirety). Furthermore, Furumatsu *et al.* have shown that spironolactone still provides substantial additive reduction of proteinuria (58% lower after 1 year) when used in non-diabetic patients already receiving dual therapy with both an ACEI plus an ARB agent, independent of any further BP changes (Furumatsu et al. (2008) Hypertens. Res. 31:59-67; herein incorporated by reference in its entirety). Prior to studies conducted during the course of developing some examples of the present disclosure of the present description, spironolactone was speculated to substantially reduce albuminuria and retard the progression of diabetic nephropathy in a significant proportion of patients who were not receiving the maximum possible protection from their ACEI or ARB agent. However, the effect of addition of spironolactone to existing ACEI or ARB treatment was unknown, especially with regard to possible adverse effects on potassium levels and GFR.

Additional anti-aldosterone agents (also known as aldosterone antagonists) include but are not limited to eplerenone, canrenone, anti-aldosterone antibodies, potassium canrenoate, carbolactones such as spirorenone, progestin analogs such as drospirenone, mesperinone and combined B-Type Natriuretic Peptide and Vasopressin-2 Receptor antagonism with Tolvaptan.

### D. Cardiac Arrythmias, Torsade de Pointes, and Sudden Death

Torsade de pointes, literally meaning twisting of points, is a distinctive form of polymorphic ventricular tachycardia (VT) characterized by a gradual change in the amplitude and twisting of the QRS complexes around the isoelectric line. Torsade de pointes (torsade) is associated with a prolonged QT interval, which may be congenital or acquired. It usually terminates spontaneously but frequently recurs and may degenerate into sustained VT and ventricular fibrillation.

Torsade is defined as a polymorphous VT in which the morphology of the QRS complexes varies from beat to beat. The ventricular rate can range from 150 beats per minute (bpm) to 250 bpm. The original report described regular variation of the morphology of the QRS vector from positive to net negative and back again. This was symbolically termed torsade de pointes, or "twisting of the point" about the isoelectric axis, because it reminded the original authors of the torsade de pointes movement in ballet. Most cases exhibit polymorphism, but the axis changes may not have regularity. The definition also requires that the QT interval be increased markedly (usually to 600 msec or greater). Cases of polymorphous VT, which are not associated with a prolonged QT interval, are treated as generic VT. Torsade usually occurs in bursts that are not sustained; thus, the rhythm strip usually shows the patient's baseline QT prolongation.

The underlying basis for rhythm disturbance is delay in phase III of the action potential. The delay is mediated by the hERG potassium channel. This prolonged period of repolarization and the inhomogeneity of repolarization times among myocardial fibers allow the dysrhythmia to emerge. The initiating electrophysiologic mechanism may be triggered activity or reentry.

Six genetic variants currently are recognized. Genotypes LQT1 and LQT2 have slow potassium channels, while LQT3 shows defects in the sodium channels. Treatment modalities soon may be based on the genotype of the individual.

The association between torsade and a prolonged QT interval has long been known, but the mechanisms involved at the cellular and ionic levels have been made clearer in approximately the last decade. The abnormality underlying both acquired and congenital long QT syndromes is in the ionic current flow during repolarization, which affects the QT interval. Various studies support the concept that prolongation of the repolarization delays the inactivation of the ion channels responsible for the inward flow of positive depolarizing currents. This leads to a further delay in repolarization and causes early after depolarization (EAD), the triggering event for torsade. The following phases are described:
- Phase 1: During initial upstroke of action potential in a normal cardiac cell, a rapid net influx of positive ions (Na⁺ and Ca⁺⁺) occurs, which results in the depolarization of the cell membrane. This is followed by a rapid transient outward potassium current (Ito), while the influx rate of positive ions (Na⁺, Ca⁺⁺) declines. This represents the initial part of the repolarization, or phase 1.
- Phase 2 is characterized by the plateau, the distinctive feature of which is the cardiac repolarization. The positive currents flowing inward and outward become almost equal during this stage.
- Phase 3 of the repolarization is mediated by activation of the delayed rectifier potassium current (IK) moving outward while the inward positive current decays. If a slow inactivation of the Ca⁺⁺ and Na⁺ currents occurs, this inward "window" current can cause single or repetitive depolarization during phases 2 and 3 (ie, EADs). These EADs appear as pathologic U waves on a surface ECG, and, when they reach a threshold, they may trigger ventricular tachyarrhythmias.

These changes in repolarization do not occur in all myocardial cells. The deep endocardial region and mid myocardial layer (composed of M cells) of the ventricle are more prone to prolongation of repolarization and EADs because they have a less rapid delayed rectifier potassium current (IKr), while other regions might have short or normal cycles. This heterogeneity of repolarization in the myocardial cells promotes the spread of triggered activity, which is initiated by EADs by a reentrant mechanism and currently is thought to be responsible for the maintenance of torsade.

Torsade is a life-threatening arrhythmia and may present as sudden cardiac death in patients with structurally normal hearts.

For both genders, the corrected QT interval is longer in persons of European descent than in persons of African descent, thus explaining the lower susceptibility to acquired torsade in persons of African descent. Females are more prone to the development of torsade than males because they have longer QT intervals. Torsade occurs in patients of a wide age range, from newborn to 86 years. If it occurs at an early age, the cause usually is due to congenital long QT syndrome. In later years, the cause usually is due to acquired long QT syndrome.

Patients with torsade usually present with recurrent episodes of palpitations, dizziness, and syncope; however, sudden cardiac death can occur with the first episode. Nausea, cold sweats, shortness of breath, and chest pain also may occur but are nonspecific and can be produced by any form of tachyarrhythmia.

Of all the diuretics, Indapamide is known to be associated with highest risk of TdP (Sander, Guillory et al. (2002) Am. J. Geriatr. Cardiol. 11:197-202; herein incorporated by reference in its entirety).

**Table 1. Action of indapamide and anti-aldosterone agent on mechanisms underlying ventricular repolarization, QT interval and Torsade**

| Electrophysiology of heart | Effect on | Effect on QT interval | Aldosterone | Anti-aldosterone | Indapamide |
|---|---|---|---|---|---|
| Inward sodium current | Slows repolarization | Prolongs QT | Increases sodium current (Boixel, Gaviltet et al. 2006) | Inhibits Na⁺ current & | No effect |
| | | | | Shortens QT | |
| Inward Calcium current | Slows repolarization | Prolongs QT | Increases calcium current | Inhibits inward calcium current | No effect |
| | | | | & | |
| | | | | Shortens QT | |
| Outward potassium current | Promotes repolarization | Shortens QT | No effect | No effect | Slows outward K+ current & |
| | | | | | Prolongs QT |
| QT interval | | | | shortened | prolongs |
| QT dispersion | | | | Reduced | increased |
| Torsade | | | | prevents | predisposes |

Indapamide has been shown to produce QT interval prolongation and torsade de pointes (TdP) in the presence of normal electrolytes (Letsas, Alexanian et al. (2006) Int. J. Cardiol. 112:373-374; herein incorporated by reference in its entirety). The major action of indapamide that leads to slowing of repolarization, prolongation of QT interval and thereby predisposition to TdP is a direct electrophysiological effect on ionic currents. In particular, indapamide blocks the slow component of the delayed rectifier K+ current (IKs) (Turgeon, Daleau et al. (1994) Circ. Res. 75:879-886; herein incorporated by reference in its entirety).

### E. Disease States and Conditions

### i. Diabetes

The present description finds use in the treatment of subjects with diabetes or a diabetes-related condition involving e.g., impaired glucose tolerance, impaired insulin sensitivity, impaired insulin production. Such conditions and disease states include diabetes mellitus, type I diabetes, type II diabetes, gestational diabetes, metabolic syndrome, metabolic syndrome X, syndrome X, insulin resistance syndrome, Reaven's syndrome, CHAOS, and malnutrition-related diabetes mellitus. Such patients are at risk for and/or experience an increased incidence and severity of renal dysfunction and renal disease, as described below.

### ii. Diabetic nephropathy

Some examples of the present disclosure of the present description find use in patients with diabetic nephropathy. Diabetic nephropathy, also known as Kimmelstiel-Wilson syndrome and intercapillary glomerulonephritis, is a progressive kidney disease caused by angiopathy of capillaries in the kidney glomeruli. It is characterized by nephrotic syndrome and diffuse glomerulosclerosis. It is due to longstanding diabetes mellitus, and is a prime indication for dialysis in many Western countries.

The syndrome can be seen in patients with chronic diabetes (usually less than 15 years after onset), so patients are usually of older age (between 50 and 70 years old). The disease is progressive and may cause death two or three years after the initial lesions, and is more frequent in men. Diabetic nephropathy is the most common cause of chronic kidney failure and end-stage kidney disease in the United States. People with both type 1 and type 2 diabetes are at risk. The risk is higher if blood-glucose levels are poorly controlled. Further, once nephropathy develops, the greatest rate of progression is seen in patients with poor control of their blood pressure. Patients with high cholesterol level in their blood are also at greater risk.

The earliest detectable change in the course of diabetic nephropathy is a thickening in the glomerulus. At this stage, the kidney may start producing more serum albumin (plasma protein) than normal in the urine (albuminuria), referred to as "microalbuminuria". As diabetic nephropathy progresses, increasing numbers of glomeruli are destroyed by nodular glomerulosclerosis. The amounts of albumin excreted in the urine increases, and may be detected by ordinary urinalysis techniques. At this stage, a kidney biopsy shows diabetic nephropathy.

Kidney failure provoked by glomerulosclerosis leads to fluid filtration deficits and other disorders of kidney function. There is an increase in blood pressure (hypertension) and fluid retention in the body plus a reduced plasma oncotic pressure causes edema. Other complications may be arteriosclerosis of the renal artery and proteinuria.

Throughout its early course, diabetic nephropathy has no symptoms. Symptoms develop in later stages and may be a result of excretion of high amounts of protein in the urine or due to renal failure. Symptoms include but are not limited to edema, anorexia, nausea, vomiting, malaise, fatigue, headache, frequent hiccups, and generalized itching. The first laboratory abnormality is a positive microalbuminuria test. Most often, the diagnosis is suspected when a routine urinalysis shows proteinuria. The urinalysis may also show glucose in the urine, especially if blood glucose is poorly controlled. Serum creatinine and BUN may increase as kidney damage progresses.

A kidney biopsy confirms the diagnosis, although it is not always necessary if the case is straightforward, with a documented progression of proteinuria over time and presence of diabetic retinopathy on examination of the retina of the eyes.

Standard treatments include administration of ACE inhibitor agents and/or ARB agents; however, combination therapy, according to the ONTARGET study (Yusuf et al. (2008) New Engl. J. Med. 358:1547-1559; herein incorporated by reference in its entirety) can worsen major renal outcomes including increasing serum creatinine and causing a greater demand in estimated GFR.

Diabetic nephropathy continues to gradually progress. Complications of chronic kidney failure are more likely to occur earlier, and progress more rapidly, when it is caused by diabetes than when it occurs due to other causes. Even after initiation of dialysis or after transplantation, people with diabetes tend to do worse than those without diabetes. Possible complications include but are not limited to hypoglycemia, rapidly progressing chronic kidney failure, end-stage kidney disease, hyperkalemia, severe hypertension, complications of hemodialysis, complications of kidney transplant, coexistence of other diabetes complications, peritonitis (if peritoneal dialysis is used), and increased infections.

### iii. Liver Failure

Some examples of the present disclosure of the present description find use in patients with liver failure. Liver failure is the inability of the liver to perform its normal synthetic and metabolic function as part of normal physiology. Two forms include acute liver failure, and chronic liver failure. In acute liver failure, hepatic encephalopathy (confusion, stupor and coma) occurs within four weeks of the first symptoms of a liver problem (such as jaundice). Chronic liver failure usually occurs in the context of cirrhosis (itself caused by factors including but not limited to excessive alcohol intake, hepatitis B, hepatitis C, or autoimmune, hereditary and metabolic causes (e.g., iron or copper overload or non-alcoholic fatty liver disease).

### iv. Hypertension

Some examples of the present disclosure of the present description find use in patients with hypertension, which may be caused by many different diseases and conditions. Hypertension is a chronic medical condition in which the blood pressure is elevated. It is also referred to as high blood pressure or shortened to HT, HTN or HPN. The word "hypertension", by itself, normally refers to systemic, arterial hypertension. Hypertension can be classified as either essential (primary) or secondary. Essential or primary hypertension indicates that no medical cause is known. Secondary hypertension indicates that the high blood pressure is a result of another condition, such as kidney disease or tumors (e.g., adrenal adenoma or pheochromocytoma). A 2003 American Heart Association classification recommends blood pressure criteria for defining normal blood pressure, prehypertension, hypertension (stages I and II), and isolated systolic hypertension, the latter being common occurrence among the elderly. These readings are based on the average of seated blood pressure readings that were properly measured during two or more office visits. In individuals older than 50 years, hypertension is considered to be present when a person's blood pressure is consistently at least 140 mmHg systolic or 90 mmHg diastolic. Various agents find use in controlling hypertension (systolic and/or diastolic hypertension); e.g., indapamide is efficacious for systolic hypertension.

### v. Heart Failure

In certain examples of the present disclosure, methods of the present description find use in the treatment of congestive heart failure (CHF), the severe manifestation of which is pulmonary edema. CHF is an imbalance in pump function in which the heart fails to adequately maintain the circulation of blood. CHF can be categorized as right sided and left sided ventricular failure. Right-sided failure causes elevated systemic venous pressure, whereas left ventricular failure is secondary to reduced forward flow into the aorta and systemic circulation. Furthermore, heart failure can be subdivided into systolic and diastolic dysfunction. Systolic dysfunction is characterized by a dilated left ventricle with impaired contractility, whereas diastolic dysfunction occurs in a normal or intact left ventricle with impaired ability to relax and receive as well as eject blood. In the New York Heart Association's functional classification of CHF, Class I describes a patient whose normal physical activity is not limited by symptoms. Class II occurs when ordinary physical activity results in fatigue, dyspnea, or other symptoms. Class III is characterized by a marked limitation in normal physical activity. Class IV is defined by symptoms at rest or with any physical activity. CHF and/or pulmonary edema may be caused by coronary artery disease (e.g., secondary to loss of left ventricular muscle), ongoing ischemia, decreased diastolic ventricular compliance, hypertension, valvular heart disease, congenital heart disease, other cardiomyopathies, myocarditis, infectious endocarditis, pregnancy, and hyperthyroidism. CHF is often precipitated by cardiac ischemia or dysrhythmias, cardiac or extracardiac infection, pulmonary embolus, physical or environmental stresses, changes or noncompliance with medical therapy, dietary indiscretion, or iatrogenic volume overload.

### vi. Edema

In certain examples of the present disclosure, methods of the present description find use in the treatment of edema, which is an abnormal accumulation of fluid beneath the skin, or in one or more cavities of the body. Causes of edema which are generalized to the whole body can cause edema in multiple organs and peripherally. For example, severe heart failure (CHF, as described *supra*) can cause pulmonary edema, pleural effusions, ascites and peripheral edema, the last of which effects can also derive from less serious causes. Organ-specific edema types include cerebral edema, pulmonary edema, pleural efflusions, corneal edema, periorbital edema, cutaneous edema, myxedema, edema of the feet, and lymphedema.

### vii. Kidney Diseases (Nephropathy)

Many diseases affect kidney function by attacking the glomeruli, the tiny units within the kidney where blood is filtered. Glomerular diseases include many conditions with a variety of genetic and environmental causes, but they fall into two major categories: glomerulonephritis, wherein there is inflammation of the membrane tissue in the kidney that serves as a filter, separating wastes and extra fluid from the blood; and glomerulosclerosis, wherein there is scarring or hardening of the tiny blood vessels within the kidney. Although glomerulonephritis and glomerulosclerosis have different causes, they can both lead to kidney failure.

Types of nephropathies include but are not limited to drug-induced nephropathy, Systemic lupus erythematosus, Goodpasture's syndrome, IgA nephropathy, Alport syndrome, acute post-streptococcal glomerulonephritis (PSGN), bacterial endocarditis, HIV-induced nephropathy, glomerulosclerosis, diabetic nephropathy, focal segmental glomerulosclerosis (FSGS), membranous nephropathy (also called membranous glomerulopathy), and minimal change disease (MCD).

Stages of kidney failure are categorized as acute renal failure, chronic kidney disease, and total kidney failure (sometimes called end-stage renal disease or ESRD).

### F. Methods of Combined Therapy

Resolution or prevention of proteinuria-associated conditions (e.g., renal dysfunction) is a major clinical challenge. Compositions and methods of the present description provide means of ameliorating this problem by effectively administering a combined therapy approach. However, it should be noted that traditional combination therapy may be employed in combination with the compositions of the present description. For example, in some examples of the present disclosure of the present description, combination therapies comprising an indoline agent and an anti-aldosterone agent may be used before, after, or in combination with traditional therapies.

To treat a subject using the methods and compositions of the present description in combination therapy, one contacts a "target" cell with the compositions described herein and at least one other agent. These compositions are provided in a combined amount effective to have a therapeutic effect the cell. This process may involve contacting the cells with multiple agent(s) or factor(s) at the same time. This may be achieved by contacting the cell with a single composition or pharmacological formulation that includes both agents, or by contacting the cell with two distinct compositions or formulations, at the same time, wherein one composition includes, for example, an expression construct and the other includes a therapeutic agent.

Alternatively, indoline (e.g., indapamide) treatment may precede or follow the second agent (e.g., an anti-aldosterone agent) by intervals ranging from minutes to weeks. In examples of the present disclosure where indoline agent (e.g., indapamide) and anti-aldosterone agent are applied separately to the cell, one would generally ensure that a significant period of time did not expire between the time of each delivery, such that the indoline agent (e.g., indapamide) and anti-aldosterone agent would still be able to exert an advantageously combined effect on the cell. In such instances, it is contemplated that cells are contacted with both modalities within about 12-24 hours of each other and, more preferably, within about 6-12 hours of each other, with a delay time of only about 12 hours being most preferred. In some situations, it may be desirable to extend the time period for treatment significantly, however, where several days (2 to 7) to several weeks (1 to 8) lapse between the respective administrations.

In some examples of the present disclosure, more than one administration of the therapeutic composition of the present description or the other agent are utilized. Various combinations may be employed, where the indoline agent (e.g., indapamide) is "A" and the anti-aldosterone agent (e.g., spironolactone) is "B", as exemplified below:
A/B/A, B/A/B, B/B/A, A/A/B, B/A/A, A/B/B, B/B/B/A, B/B/A/B,
A/A/B/B, A/B/A/B, A/B/B/A, B/B/A/A, B/A/B/A, B/A/A/B, B/B/B/A,
A/A/A/B, B/A/A/A, A/B/A/A, A/A/B/A, A/B/B/B, B/A/B/B, B/B/A/B.

Other combinations are contemplated. Again, to achieve desired therapeutic effect, both agents are delivered to a cell in a combined amount effective to promote desired therapeutic outcome (e.g., decreased proteinuria, lowered blood pressure).

In some examples of the present disclosure of the description, one or more compounds of the description and an additional active agent are administered to a subject, more typically a human, in a sequence and within a time interval such that the compound can act together with the other agent to provide an enhanced benefit relative to the benefits obtained if they were administered otherwise. For example, the additional active agents can be co-administered by co-formulation, administered at the same time or administered sequentially in any order at different points in time; however, if not administered at the same time, they should be administered sufficiently close in time so as to provide the desired therapeutic or prophylactic effect. In some examples of the present disclosure, the compound and the additional active agents exert their effects at times which overlap. Each additional active agent can be administered separately, in any appropriate form and by any suitable route. In other examples of the present disclosure, the compound is administered before, concurrently or after administration of the additional active agents.

In various examples, the compound and the additional active agents are administered less than about 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, no more than 24 hours apart or no more than 48 hours apart. In other examples, the compound and the additional active agents are administered concurrently. In yet other examples, the compound and the additional active agents are administered concurrently by co-formulation.

In other examples, the compound and the additional active agents are administered at about 2 to 4 days apart, at about 4 to 6 days apart, at about 1 week part, at about 1 to 2 weeks apart, or more than 2 weeks apart.

In certain examples, indoline agent (e.g., indapamide) and an anti-aldosterone agent (and optionally additional active agents) are cyclically administered to a subject. Cycling therapy involves the administration of a first agent for a period of time, followed by the administration of a second agent and/or third agent for a period of time and repeating this sequential administration. Cycling therapy can provide a variety of benefits, e.g., reduce the development of resistance to one or more of the therapies, avoid or reduce the side effects of one or more of the therapies, and/or improve the efficacy of the treatment.

In other examples, one or more compounds of some examples of the present disclosure of the present description and optionally an additional active agent are administered in a cycle of less than about 3 weeks, about once every two weeks, about once every 10 days or about once every week. One cycle can comprise the administration of a first active agent and optionally the second active agent by infusion over about 90 minutes every cycle, about 1 hour every cycle, about 45 minutes every cycle, about 30 minutes every cycle or about 15 minutes every cycle. Each cycle can comprise at least 1 week of rest, at least 2 weeks of rest, at least 3 weeks of rest. The number of cycles administered is from about 1 to about 12 cycles, more typically from about 2 to about 10 cycles, and more typically from about 2 to about 8 cycles.

Courses of treatment can be administered concurrently to a subject, i.e., individual doses of the additional active agents are administered separately yet within a time interval such that the inventive compound can work together with the additional active agents. For example, one component can be administered once per week in combination with the other components that can be administered once every two weeks or once every three weeks. In other words, the dosing regimens are carried out concurrently even if the therapeutics are not administered simultaneously or during the same day.

### G. Dosages

The present description is not limited to particular dosages of an indoline agent (e.g., indapamide) and an anti-aldosterone agent (e.g., spironolactone). Some examples of the present disclosure of combination therapy dosage forms are illustrated in Tables 2. In some examples of the present disclosure, combinations of anti-aldosterone agents with up to 1.25 mg of indapamide are primarily indicated for the treatment of particular disease states and/or conditions including but not limited to hypertension without significant renal disease or heart failure; ascites associated with liver failure; and/or mild heart failure. In some examples of the present disclosure, combinations of anti-aldosterone agents with 2.5-5 mg indapamide are used for treatment of diseases and/or conditions including but not limited to edema or hypertension associated with stage 3-5 chronic kidney disease, and/or edema or hypertension associated with moderate or severe heart failure.

**Table 2. Exemplary dosages of spironolactone or eplerenone in combination with exemplary doses of indapamide.**

| **Spironolactone or Eplerenone (mg)** | **Indapamide (mg)** |
|---|---|
| | 0.5 |
| 12.5 or | 0.625 |
| 25 or | 1.0 |
| 37.5 or | 1.25 |
| 50 or | 1.5 |
| 75 or | 2.0 |
| 100 | 2.5 |
| | 3.0 |

### Examples

The following examples are provided in order to demonstrate and further illustrate certain preferred examples of the present disclosure and aspects of the present description and are not to be construed as limiting the scope thereof.

### Example 1

### Anti-Proteinuric Action of Indapamide in Combination with an Anti-aldosterone Agent

The following case studies were observed at the Diabetes and Nephrology specialty clinics at the Martin Luther King Jr. - Multi-Service Ambulatory Clinic in South Los Angeles, CA, USA.

A 67 year old Hispanic male presented with diabetic nephropathy and hypertension. The patient was treated with 100 mg losartan, metoprolol 75 mg and amlodipine 10 mg daily. The patient had 1800 mg albuminuria per 1 gram of creatinine. The patient was treated with a combination of 25 mg spironolactone and 1.25 mg indapamide with a decline in albuminuria to 930 mg albumin per 1 gram of creatinine, a reduction of 48%.

A 46 year old Hispanic male presented with chronic kidney disease with an estimated GFR of 32 ml/min, secondary to diabetic nephropathy and hypertension, associated with heavy proteinuria. Baseline urinary albumin was 8 grams per 1 gram of creatinine, while the patient was being treated with 100 mg losartan daily. The patient was further treated with a combination of 1.25 mg indapamide and 25 mg spironolactone which led to a decline of 57.5 % in albuminuria to 3.4 grams albumin per 1 gram of creatinine over a period of 8 months.

A 57 year old Hispanic male with cirrhosis, edema and chronic hypotension (systolic blood pressure 90-100 mm of Hg) had developed chronic kidney disease secondary to diabetic nephropathy. The patient had an estimated GFR of 27 ml/min and 1970 mg albumin in urine per 1 gram creatinine. After 9 months of daily treatment with half of a 1.25 mg tablet of indapamide in combination with 25 mg spironolactone, the albuminuria was reduced to 566 mg albumin per 1 gram of creatinine, a reduction of 71 %. The reduction in proteinuria occurred even though blood pressure and GFR remained unchanged.

A 61 year old Filipina presented with diabetic nephropathy and hypertension. The patient was treated with 20 mg benazapril and 240 mg extended release diltiazem daily. The patient had 418 mg albuminuria per 1 gram of creatinine. The patient was treated with a combination of 25 mg spironolactone and 0.625 mg indapamide and albuminuria decreased to 82 mg albumin per 1 gram of creatinine, a reduction of 80%.

A 44 year old Hispanic male with alcoholic liver disease was on long-standing treatment with 200 mg spironolactone daily. The patient presented with hypertension and chronic proteinuric and hematuric kidney disease secondary to chronic glomerulonephritis, likely IgA nephropathy. The patient had albuminuria of 2606 mg per 1 gram creatinine. For hypertension and edema 2.5 mg indapamide daily was started. After 1 month of treatment, albuminuria had decreased to 1081 mg per 1 gram of creatinine, a reduction of 58.5%, even though the patient had a blood pressure of 160/100 mm of Hg.

A 64-year-old Hispanic female with diabetic nephropathy, had 1346 mg albuminuria per gram creatinine while taking lisinopril 40 mg, verapamil ER 240 mg and indapamide 0.625 mg. Spironolactone 25 mg was added and within 2 months, her albuminuria decreased to 258 mg per gram creatinine, a reduction of 81%.

The above cases are representative of the anti-proteinuric action of a combination of an anti-aldosterone agent with indapamide. The salutary effect was often seen even when the systemic blood pressure continued to stay elevated. While the present description is not limited to any particular mechanism, and an understanding of the mechanism is not necessary to practice the present description, it is contemplated that in some examples of the present disclosure, combination therapies of the present description exert local hemodynamic or direct glomerular effect rather than systemic effects contributing to the anti-proteinuric effect of combination therapy.

### Example 2

### The Thiazide-like Diuretic Metolazone in Combination with Spironolactone Does Not Reduce Proteinuria

Two thiazide-like diuretics that are effective in patients with stage 4-5 chronic kidney disease are metolazone and indapamide. During the course of developing some examples of the present disclosure of the present description, it was unexpectedly found that indapamide in combination with spironolactone, but not metolazone in combination with spironolactone, was capable of reducing proteinuria. The following case study demonstrates the failure of cotherapy with metolazone and spironolactone to reduce proteinuria.

A 62 year old Hispanic male presented with stage 4 chronic kidney disease secondary to diabetic nephropathy. The patient's urinary albumin ranged from 3.5 to 4.1 g per gram of creatinine for the past 2 years while he was treated with ACEI and pentoxifylline. Over 2 months of treatment with a combination of spironolactone 25 mg and metolazone 2.5 mg failed to reduce albuminuria. The urine albumin/creatinine ratio at conclusion of treatment was 4.3.

### Example 3

### Mitigation of Hyperuricemia by Combination of Indapamide and Spironolactone

Case 1. A 77 year old Nigerian male had a history of diabetes and hypertension for 5 years. The following is the chronological clinical and laboratory profile.

Day 0: serum potassium 5.2 mmol/L, creatinine 2.3 mg/dL, blood urea nitrogen 46 mg/dL, glycosylated hemoglobin 8.2%, LDL 143.

Day 13: the patient was seen in the Clinic with a history of nocturia. The patient was diagnosed with Stage 4 chronic kidney disease (estimated GFR 30 ml per minute) and the cause of kidney disease was considered to be excessive intake of non-steroidal anti-inflammatory drugs in the past leading to chronic tubulo-interstitial disease and renal scarring. Presence of microalbuminuria [urine albumin to creatinine ratio = 91 µg/mg (normal < 30 µg/mg)] raised the possibility of underlying diabetic nephropathy. Patient was on treatment with long acting nifedipine: 60 mg in morning, 30 mg at bedtime and hydrochlorthiazide 25 mg in the morning for hypertension. Patient had a pulse rate of 95 per minute, blood pressure 142/82 mm of Hg, body weight 74.4 kg, and pedal edema. Nifedipine and hydrochlorthiazide were discontinued. Patient was started on indapamide 2.5 mg every morning, losartan 50 mg daily and terazosin 2 mg at bedtime.

Day 25: laboratory studies revealed serum potassium 4.1 mmol/L, creatinine 2.6 mg/dL, urea nitrogen 36 mg/dL, uric acid 7.5 mg/dL.

Day 34: Pedal edema decreased, pulse 58 per min, blood pressure 150/90 mm of Hg, body weight 74.2 kg. Spironolactone 12.5 mg every morning was added.

Day 62: Blood urea nitrogen 46 mg/dL, serum creatinine 2.6 mg/dL, potassium 4.8 mmol/L, uric acid 6 mg/dL. Urine microalbumin to creatinine ratio was 35 µg/mg, a reduction of 61% from baseline 6 weeks ago.

Day 69: Body weight 73 kg. Pulse 72 per minute, blood pressure 147/86 mm of Hg. Patient was clinically improved and edema had decreased further.

Case 2. A 43 year old African American male with type II diabetes mellitus, diabetic nephropathy, albuminuria, hypercholesterolemia, hypertension was seen at a primary care clinic with uncontrolled hypertension (147/84 mm of Hg) and edema (Day 0). Patient was on treatment with felodipine 10 mg daily and atenolol 100 mg daily.

2 months: The patient was started on benazapril 10 mg and losartan 50 mg daily. Blood urea nitrogen 19 mg/dL, serum creatinine 19 mg/dL, uric acid 8.7 mg/dL, urine albumin to creatinine ratio 1220 mg/gram.

3 months: the BP was 131/72.

3.5 months: The patient was seen by a nephrologist. Blood pressure was 125/81 mm of Hg. Benazapril was discontinued.

4 months: potassium 4.0, glucose 118, TG 340, LDL 133

5 months: Blood pressure was 168/108 mm Hg. Atenolol was discontinued. Patient was started on long acting diltiazem 300 mg daily, losartan was increased to 100 mg daily and 1.25 mg indapamide daily was added.

6 months: Blood pressure was 145/93 mm Hg, spironolactone 25 mg was started.

7.5 months: EKG: QTc 441 ms, left anterior fascicular block; potassium 4.4.

9.5 months: Blood pressure was 138/79 mm Hg. There was no edema noted. Potassium 4.3, uric acid 8.2 mg/dL, blood urea nitrogen 19, creatinine 1.5, urine albumin to creatinine ratio 326 mg/gram, a decrease of 73%. Notably, despite the addition of indapamide, usually associated with a significant increment in uric acid, there had been a decrement of 0.5 mg/dL from the baseline value.

Case 3. A 60 year old Hispanic male presenting with diabetic nephropathy, hypertension and estimated GFR of 27 ml/min had asymptomatic hyperuricemia with a baseline serum uric acid level of 10.3 mg/dL. The patient was being treated with losartan 100 mg per day for hypertension and proteinuria. The patient was started on indapamide 1.25 mg and spironolactone 25 mg per day for optimal control of blood pressure. After 3 months, serum uric acid ranged between 6.4-7.2 mg/dL, a decline of 30% to 38%.

Case 4. A 56 year old Hispanic female with diabetic nephropathy, hypertension and bifascicular block presented with following baseline serum values: uric acid 7.5 mg/dL, creatinine 1.4 mg/dL, SUN 36 mg/dL, urine albumin to creatinine ratio 1015 mg/g. Patient was being treated with losartan 100 mg and amlodipine 5 mg per day for hypertension and proteinuria. Patient was started on Indapamide 1.25 mg per day for hypertension at time 0. After 9 months of treatment, serum creatinine and urea nitrogen were unchanged and serum uric acid was 7.0 mg/dL. Two weeks later, patient was started on spironolactone 25 mg per day and amlodipine dose was increased to 10 mg per day for optimal control of blood pressure. After 2 weeks of such treatment, serum creatinine, urea nitrogen were largely unchanged but serum uric acid was 6.1 mg/dL, a decline of about 13%. With the combination of indapamide and spironolactone, the urine albumin to creatinine ratio was reduced to 140 mg/g, a decline of 86%.

### Example 4

### Clinical Study of Indapamide plus Spironolactone Combination Therapy Versus Thiazide Diuretic for Additive Control of Albuminuria in Diabetic Nephropathy

A 6-month, prospective, open-label, randomized pilot study is described to compare the efficacy of the indapamide-spironolactone combination versus hydrochlorothiazide (HCTZ), when added to angiotensin-converting enzyme inhibitor (ACEI) or angiotensin receptor blocker (ARB) therapy, for reducing proteinuria while targeting a uniform blood pressure (BP) goal, across a wide spectrum of diabetic subjects with confirmed micro- or macroalbuminuria. Subjects will be recruited from the Diabetes and Nephrology specialty clinics at the Martin Luther King Jr. - Multi-Service Ambulatory Clinic in South Los Angeles, CA, USA. MLK-MACC serves a large community of low-income and low-educational level individuals in the inner city of South Los Angeles, consisting of approximately 70% Hispanic and 25% African-Americans. Male or female adult subjects with type 1 or type 2 diabetes and confirmed evidence of micro- or macroalbuminuria (30 mg per g urine creatinine or higher on at least two consecutive determinations), who are already being treated with an ACE inhibitor or ARB agent, will be enrolled.

### Selected Inclusion / Exclusion Criteria:

### Inclusion:

- Age 18 to 70 inclusive
- Diagnosed type 1 or type 2 diabetes mellitus
- Confirmed microalbuminuria (≥ 30 mg per gram of urinary creatinine) or macroalbuminuria (≥ 300 mg per gram of urinary creatinine) on at least two serial determinations performed a minimum of 1 week and a maximum of 6 months apart
- Previous therapy with a stable dose (i.e., no dose change for at least 1 month) of either an ACEI or ARB agent
- Baseline systolic BP ≥ 130 mm Hg or diastolic BP ≥ 80 mm Hg
- Estimated GFR of at least 30 mL/min
- HbA_{1c} level < 10.0 %

### Exclusion:

- Subjects with any contraindications or known intolerances to therapy with *both* ACEI and ARB agents;
- Subjects with contraindications or intolerance to treatment with thiazides, indapamide, or spironolactone
- BP averaging ≥ 160 mm Hg systolic or ≥ 100 mm Hg diastolic
- Subjects with symptoms of postural hypotension on their current dose of ACEI or ARB
- Known history or clinical suspicion of renal dysfunction attributable to causes other than diabetic or hypertensive nephropathy (e.g., glomerulonephritis, obstructive uropathy, etc.)
- Serum potassium level < 3.0 mEq/L, or > 6.0 mEq/L; sodium level > 150 mEq/L, calcium level > 11.5 mg/dL, a history of active gout, frequent gout attacks, or uric acid level > 10 mg/dL
- Subjects currently taking indapamide, thiazide or potassium-sparing diuretics that cannot be safely and effectively washed out to provide a reliable baseline assessment of ACR

### Outcomes:

*Primary Outcome:* Change from baseline in albumin-creatinine ratio (ACR), expressed as the ratio of mg of urinary protein per gram of urinary creatinine, obtained from a morning urine sample
*Secondary Outcomes:* Change from baseline in:
- Systolic and diastolic blood pressures
- Serum creatinine, and estimated GFR (eGFR) based on both the MDRD equation and serum cystatin C levels.
- Serum Na⁺, K⁺, Ca⁺⁺, uric acid levels
- Highly-sensitive C-reactive protein (hsCRP), interleukin-6 (IL-6), oxidized LDL (oxLDL)
- Clinical adverse events
*Other Variables:* Body mass index (BMI); HbA_{1c}; LDL-cholesterol; medication compliance (as determined by pill counts)

### Study Procedures:

*Baseline Assessment:* Screening will include a medical history, vital signs and anthropometry, CBC, complete chemistry panel (including electrolytes, BUN, serum creatinine, estimated GFR based on the MDRD equation and serum cystatin C, hepatic transaminases, calcium, uric acid), HbA_{1c}, fasting lipid profile, routine urinalysis, and a determination of urinary albumin-creatinine ratio (ACR) based on a spot urine sample. Subjects determined to be eligible based on this screening should have a repeat ACR performed within 1 week (or previously had an ACR determination performed within the past 6 months) for confirmation.

*Run-In Phase:* To minimize the potential influence that severe hyperglycemia or hyperlipidemia may have on ACR, all qualifying subjects will pass through a run-in phase of up to 3 months in duration, the aim of which is to improve glycemic and lipid control according to recommended guidelines. Similarly, hyperlipidemia has been implicated as contributing to renal dysfunction, as a consequence of an atherosclerotic and inflammatory process damaging the glomeruli and tubulo-interstitium. Therefore, the run-in period will target: 1) Glucose control to at least < 8.0%, using dietary and lifestyle modifications plus oral agents and/or insulin; and 2) Control of LDL-cholesterol to < 100 mg/dL using dietary and lifestyle modifications plus HMG-CoA reductase inhibitor agents (statins) and/or ezetimibe. In addition, the run-in period will also be an opportunity to emphasize compliance with the subject's concurrent therapies, including their ACEI or ARB therapy. Up to 3 months will be permitted to achieve these goals, and the frequency of follow-up required to titrate therapy to meet these goals will be at the discretion of the investigator. Subjects who already meet these goals reliably may proceed to the baseline visit and randomization. Subjects who fail to meet both of these goals reliably after 3 months will be withdrawn, but may be re-screened at a later date if they can meet both goals at that time.

*Randomization:* Qualifying subjects who pass the run-in period will be randomized to receive either the indapamide and spironolactone combination or HCTZ, added to their previous therapy.

*Study Medications, Dose Titration, and Rescue Therapy:* Because all study medications in this open-label trial are FDA-approved for the management of hypertension, the subject will be given individual prescriptions (without refills). sufficient for 3 months at baseline and at 2-month intervals. All unused pills must be presented to the study coordinators and counted at each of the 2, 4, and 6-month visits to determine compliance, and an adequate supply for an additional 3-months will be provided at each of these visits to ensure the subject has an adequate supply. The subject's compliance with his/her pre-study ACEI or ARB agents and all other concurrent therapies will also be reviewed at these visits. The dose of all other anti-hypertensive agents should remain constant throughout the duration of the study, unless a dose reduction is needed to avoid symptoms of postural hypotension. A tiered, dose-titration algorithm for their assigned study medications will be applied for all subjects. Treatment will be initiated with Tier 1 doses upon randomization. If BP remains ≥ 130/80 mm Hg at the Month 1 visit, subjects will be increased to the Tier 2 doses. For subjects randomized to the indapamide-spironolactone group, Tier 1 will consist of indapamide 1.25 mg once daily together with spironolactone 25 mg once daily, and Tier 2 will consist of indapamide 2.5 mg once daily together with spironolactone 25 mg once daily. For subjects randomized to the thiazide group, Tier 1 will consist of HCTZ 12.5 mg once daily, and Tier 2 will consist of HCTZ 25 mg once daily. If BP remains ≥ 130/80 mm Hg at the Month 2 visit after treatment with the Tier 2 dose, subjects will be additionally prescribed clonidine, starting at 0.1 mg bid, titrating up to 0.3 mg bid, as tolerated; and/or guanfacine, starting at 1 mg qhs, titrating up to 3 mg qhs, as tolerated; until BP is controlled to < 130/80 mm Hg. Dose titrations of clonidine and/or guanfacine may occur more frequently than the scheduled visits, if necessary. Compliance with all other concurrent anti-hypertensive agents will be reinforced at each visit. If a BP < 130/80 mm Hg cannot be achieved on the Tier 2 doses plus maximally tolerated doses of clonidine and/or guanfacine (and medication compliance is adequate), the subject will be withdrawn from the study. Once a BP of < 130/80 mm Hg is reliably achieved, doses of all anti-hypertensive medications will be held constant from that point forth for the remainder of the study, unless symptoms of postural hypotension occur (see below). Thereafter, if the BP rises again to ≥ 130/80 mm Hg at any time for reasons other than poor medication compliance, intensive counseling will be applied with respect to dietary sodium restriction and weight control through non-pharmacological measures. If the average BP should rise to >140/90 mm Hg for reasons that are not readily remediable (e.g., poor medication compliance) or that cannot be identified, the subject will be withdrawn from the study.

If at any time during the study, subjects experience symptoms of postural hypotension (especially if corroborated by a systolic BP < 100 mm Hg) for reasons that are not readily remediable (e.g., medication administration errors), dose reductions of anti-hypertensive medications will conform to the following sequence: 1) Taper the doses of any centrally-acting agent(s) (i.e., clonidine and/or guanfacine) or any concurrent calcium channel blocker agent, until symptoms of postural hypotension are eliminated; 2) If symptoms persist despite discontinuation of those agents, for subjects without a history of peripheral edema or CHF, lower the study medication dosage by one Tier at a time; or for subjects with a history of peripheral edema or CHF, lower the dose of the subject's ACEI or ARB agent by one half, until symptoms of postural hypotension are eliminated; 3) If symptoms persist despite complete discontinuation of the subject's study medications or ACEI/ARB agent, the subject should be withdrawn from the study.

| **Table 3. Summary of Study Procedures** | | | | | | | |
|---|---|---|---|---|---|---|---|
| | **Screen** | **Run-In ^{a}** | **Baseline ^{b}** | **Month** | | | |
| | | | | **1** | **2** | **4** | **6** |
| **Medical History** | **x** | | | | | | |
| **Vital Signs (incl. BP), Weight, Height** | **x** | **x** | **x** | **x** | **x** | **x** | **x** |
| **Randomization** | | | **x** | | | | |
| **Chemistry Panel, Urinalysis, eGFR, Cystatin C** | **x** | **x** | **x** | **x** | **x** | **x** | **x** |
| **HbA_{1c}, Lipid Profile** | **x** | **x** | **x** | | **x** | **x** | **x** |
| **ACR, Inflammatory Markers** | **x** | | **x** | | **x** | **x** | **x** |
| **Dietary Counseling, Review Adverse Events** | | **x** | **x** | **x** | **x** | **x** | **x** |
| **Urine Pregnancy Test ^{c}** | **x** | | **x** | | **x** | **x** | **x** |
| **Dispense Study Medications** | | | **x** | | **x** | **x** | |
| **Review Study Medications and Pill Counts** | | | | **x** | **x** | **x** | **x** |

| | | | | | | | |
|---|---|---|---|---|---|---|---|
| ^{a} Frequency of visits and laboratory tests during the run-in period may be scheduled at the investigator's discretion, depending on the severity of glucose and lipid elevations; the run-in period should not exceed 3 months in total. ^{b} For subjects who do not require the run-in period, parameters obtained at screening may substitute as the baseline measurements. ^{c} Only for women of childbearing potential. | | | | | | | |

### Data Analysis:

Data will be analyzed according to both an intent-to-treat (ITT) paradigm and a per-protocol (PP) paradigm consisting of completed subjects only. For the ITT analyses, data from randomized subjects who fail to complete the study in its entirety will be imputed using a last-value-carried-forward (LVCF) approach based on their last known post-randomization values. Non-normal variables will be transformed using an appropriate method or analyzed using equivalent non-parametric tests.

Analyses of the study's key outcome parameters (ACR, systolic and diastolic BP, creatinine, eGFR using both methods, electrolytes, and clinical adverse events) will each be analyzed by repeated measures analysis of covariance (ANCOVA) to determine the between-group differences over time (group x time interaction, corrected for baseline), both as unadjusted analyses and after adjustment for the variables of age, gender, self-reported ethnicity (Hispanic vs. African-American vs. Other), changes in BMI, changes in HbA_{1c}, changes in LDL-cholesterol, and most importantly, the changes in systolic and diastolic BP. Bias due to variations in BP response will be reduced by the fact that the protocol targets all subjects to the same BP goal (i.e., <130/80 mm Hg), but results will still be adjusted for BP to account for any influences within the normotensive range.

In addition, subjects will be stratified into subgroups based on: a) gender; b) ethnicity (African-Americans vs. Hispanics); c) microalbuminuria vs. macroalbuminuria (above vs. below 300 mg/gram creatinine); d) eGFR (above vs. below 60 mL/min); and e) baseline BP (above vs. below 130/80 mm Hg); and then analyzed separately to determine if any between-group differences in the study's key outcomes are more or less pronounced in these specific subgroups. All multivariate adjustments will be conducted using an appropriate linear regression model. Statistical significance will be p<0.05.

## Claims

1. Use of indapamide for the preparation of a medicament for co-administration with an anti-aldosterone agent for treatment of a condition in a subject, wherein the anti-aldosterone agent is spironolactone or epleronone and said condition is proteinuria, albuminuria or hyperuricemia.

2. Use of an anti-aldosterone agent for the preparation of a medicament for co-administration with indapamide for treatment of a condition in a subject, wherein the anti-aldosterone agent is spironolactone or epleronone and said condition is proteinuria, albuminuria or hyperuricemia.

3. A composition comprising indapamide and an anti-aldosterone agent, wherein the anti-aldosterone agent is spironolactone or epleronone.

4. The use according to any one of claims 1 and 2, wherein indapamide and said anti-aldosterone agent are formulated for simultaneous administration in a single delivery vehicle.

5. The use according to claim 4, wherein said single delivery vehicle is formulated for oral administration.

6. The use according to any one of claims 1 or 2, wherein indapamide and said anti-aldosterone agent are provided by more than one delivery vehicle.

7. The use according to any one of claims 1, 2, 4 or 6, wherein the medicament further comprises an agent having a function selected from the group consisting of preventing or reducing proteinuria, promoting natriuresis, and preventing or reducing hypertension.

8. The use according to claim 7, wherein said agent is selected from the group consisting of an angiotensin converting enzyme inhibitor, an anti-renin agent, an endothelin antagonist, a calcium channel blocker, a beta blocker, a nitric oxide donor, a ganglionic blocker, an alpha-methyl dopa, an alpha blocker and an anti-inflammatory agent.

## Patentansprüche

1. Verwendung von Indapamid für die Herstellung eines Medikaments zur gemeinsamen Verabreichung mit einem Anti-Aldosteron-Mittel zur Behandlung einer Erkrankung bei einer Zielperson, wobei das Anti-Aldosteron-Mittel Spironolacton oder Eplerenon ist, und wobei die Erkrankung Proteinurie, Albuminurie oder Hyperurikämie ist.

2. Verwendung eines Anti-Aldosteron-Mittels für die Herstellung eines Medikaments zur gemeinsamen Verabreichung mit Indapamid zur Behandlung einer Erkrankung bei einer Zielperson, wobei das Anti-Aldosteron-Mittel Spironolacton oder Eplerenon ist, und wobei die Erkrankung Proteinurie, Albuminurie oder Hyperurikämie ist.

3. Zusammensetzung, welche Indapamid und ein Anti-Aldosteron-Mittel umfasst, wobei das Anti-Aldosteron-Mittel Spironolacton oder Eplerenon ist.

4. Verwendung nach einem der Ansprüche 1 und 2, wobei Indapamid und das Anti-Aldosteron-Mittel zur gleichzeitigen Verabreichung in einer einzigen Bereitstellungsträgersubstanz bereitgestellt werden.

5. Verwendung nach Anspruch 4, wobei die einzige Bereitstellungsträgersubstanz so gestaltet ist, dass sie zur oralen Verabreichung dient.

6. Verwendung nach einem der Ansprüche 1 oder 2, wobei Indapamid und das Anti-Aldosteron-Mittel durch mehr als eine Trägersubstanz bereitgestellt werden.

7. Verwendung nach einem der Ansprüche 1, 2, 4 oder 6, wobei das Medikament außerdem einen Wirkstoff beinhaltet, welcher eine Funktion hat, die ausgewählt ist aus einer Gruppe, die das Verhindern oder Verringern von Proteinurie, die Förderung von Natriurese und das Verhindern oder Reduzieren von Hypertension umfasst.

8. Verwendung nach Anspruch 7, wobei der Wirkstoff ausgewählt ist aus einer Gruppe, die Angiotensin-Konversions-Enzym-Hemmer, Anti-Renin-Mittel, Endothelin-Antagonisten, Calciumkanalblocker, Betablocker, Stickoxiddonoren, Ganglienblocker, Alpha-Methyl-Dopa, Alphablocker und entzündungshemmende Mittel umfasst.

## Revendications

1. Utilisation d'indapamide pour la préparation d'un médicament pour la co-administration avec un agent anti-aldostérone pour le traitement d'une affection chez un sujet, dans laquelle l'agent anti-aldostérone est la spironolactone ou l'épléronone et ladite affection est une protéinurie, une albuminurie ou une hyperuricémie.

2. Utilisation d'un agent anti-aldostérone pour la préparation d'un médicament pour la co-administration avec l'indapamide pour le traitement d'une affection chez un sujet, dans laquelle l'agent anti-aldostérone est la spironolactone ou l'épléronone et ladite affection est une protéinurie, une albuminurie ou une hyperuricémie.

3. Composition comprenant l'indapamide et un agent anti-aldostérone, dans laquelle l'agent anti-aldostérone est la spironolactone ou l'épléronone.

4. Utilisation selon l'une quelconque des revendications 1 et 2, dans laquelle l'indapamide et ledit agent anti-aldostérone sont formulés pour une administration simultanée dans un véhicule de distribution unique.

5. Utilisation selon la revendication 4, dans laquelle ledit véhicule de distribution unique est formulé pour une administration par voie orale.

6. Utilisation selon l'une quelconque des revendications 1 ou 2, dans laquelle l'indapamide et ledit agent anti-aldostérone sont fournis par plus d'un véhicule de distribution.

7. Utilisation selon l'une quelconque des revendications 1, 2, 4 ou 6, dans laquelle le médicament comprend en outre un agent présentant une fonction sélectionnée à partir du groupe constitué par la prévention ou la réduction d'une protéinurie, le renforcement d'une natriurèse, et la prévention ou la réduction d'une hypertension.

8. Utilisation selon la revendication 7, dans laquelle ledit agent est sélectionné à partir du groupe constitué par un inhibiteur de l'enzyme de conversion de l'angiotensine, un agent anti-rénine, un antagoniste de l'endothéline, un inhibiteur des canaux calciques, un bêta-bloquant, un donneur d'oxyde nitrique, un ganglioplégique, un alpha-méthyldopa, un alpha-bloquant et un agent anti-inflammatoire.
